# EUROPEAN PATENT SPECIFICATION

(11) **EP 2 485 739 B1**
(45) Date of publication and mention of the grant of the patent: **10.12.2014**
(21) Application number: 10822331.4
(22) Date of filing: 08.10.2010
(51) Int. Cl.: A61K 31/7105, A61K 39/395, A61K 38/00, A61P 41/00

(54) **METHODS AND COMPOSITIONS FOR MAINTENANCE OF A FUNCTIONAL WOUND**
VERFAHREN UND ZUSAMMENSETZUNGEN ZUR MODULATION EINER FUNKTIONELLEN WUNDE
MÉTHODES ET COMPOSITIONS DE MODULATION DE CICATRISATION DE PLAIE FONCTIONNELLE

(30) Priority: 09.10.2009 US 250006 P
(43) Date of publication of application: 15.08.2012
(73) Proprietor: Singapore Health Services PTE. Ltd., Singapore 169611 (SG)
(72) Inventor: WONG, Tina, Singapore 168751 (SG); SEET, Li-Fong, Singapore 168751 (SG)
(74) Representative: Viering, Jentschura & Partner Patent- und Rechtsanwälte
(86) International application number: PCT/SG2010/000382
(87) International publication number: WO 2011/043739

(56) References cited:
- WO-A1-2010/065969
- WO-A1-2011/130464
- US-A1- 2009 220 589
- BASU, A. ET AL.: 'Impaired wound healing in mice deficient in a matricellular protein SPARC (osteonectin, BM-40)' BMC CELL BIOLOGY vol. 2, August 2001, page 15, XP021014032
- ZHOU, X. ET AL.: 'Small Interfering RNA Inhibition of SPARC Attenuates the Profibrotic Effect of Transforming Growth Factor beta1 in Culture Normal Human Fibroblasts' ARTHRITIS & RHEUMATISM vol. 52, no. 1, January 2005, pages 257 - 261, XP008161884
- ZHOU, X. ET AL.: 'Attenuation of Collagen Production With Small Interfering RNA of SPARC in Culture Fibroblasts From the Skin of Patients With Scleroderma' ARTHRITIS & RHEUMATISM vol. 54, no. 8, August 2006, pages 2626 - 2631, XP008161896
- CAMINO, A. M. ET AL.: 'Adenovirus-mediated inhibition of SPARC attenuates liver fibrosis in rats' JOURNAL OF GENE MEDICINE vol. 10, no. 9, September 2008, pages 993 - 1004, XP008161886
- CHUA, J. ET AL.: 'Increased SPARC expression in primary angle closure glaucoma iris' MOLECULAR VISION vol. 14, October 2008, pages 1886 - 1892, XP008161894
- HADDADIN, R. I. ET AL.: 'SPARC-null Mice Exhibit Lower Intraocular Pressures' vol. 50, no. 8, August 2009, pages 3771 - 3777, XP008161887
- SEET, L-F. ET AL. SPARC DEFICIENCY RESULTS IN IMPROVED SURGICAL SURVIVAL IN A NOVEL MOUSE MODEL OF GLAUCOMA FILTRATION SURGERY vol. 5, no. 2, February 2010, pages E9415 - 1-12, XP008161890

### SEQUENCE LISTING

<110> SINGAPORE HEALTH SERVICES PTE. LTD.
<120> METHODS AND COMPOSITIONS FOR MAINTENANCE OF A FUNCTIONAL WOUND
<130> P48985
<140> 10822331.4 <141> 2010-10-08
<150> US61/250,006
   <151> 2009-10-09
<160> 11
<170> PatentIn version 3.5
<210> 1
   <211> 303
   <212> PRT
   <213> Homo sapiens
<400> 1
   Met Arg Ala Trp Ile Phe Phe Leu Leu Cys Leu Ala Gly Arg Ala Leu
   1               5                   10                  15
   Ala Ala Pro Gln Gln Glu Ala Leu Pro Asp Glu Thr Glu Val Val Glu
               20                  25                  30
   Glu Thr Val Ala Glu Val Thr Glu Val Ser Val Gly Ala Asn Pro Val
           35                  40                  45
   Gln Val Glu Val Gly Glu Phe Asp Asp Gly Ala Glu Glu Thr Glu Glu
       50                  55                  60
   Glu Val Val Ala Glu Asn Pro Cys Gln Asn His His Cys Lys His Gly
   65                  70                  75                  80
   Lys Val Cys Glu Leu Asp Glu Asn Asn Thr Pro Met Cys Val Cys Gln
                   85                  90                  95
   Asp Pro Thr Ser Cys Pro Ala Pro Ile Gly Glu Phe Glu Lys Val Cys
               100                 105                 110
   Ser Asn Asp Asn Lys Thr Phe Asp Ser Ser Cys His Phe Phe Ala Thr
           115                 120                 125
   Lys Cys Thr Leu Glu Gly Thr Lys Lys Gly His Lys Leu His Leu Asp
       130                 135                 140
   Tyr Ile Gly Pro Cys Lys Tyr Ile Pro Pro Cys Leu Asp Ser Glu Leu
   145                 150                 155                 160
   Thr Glu Phe Pro Leu Arg Met Arg Asp Trp Leu Lys Asn Val Leu Val
   165 170 175
   Thr Leu Tyr Glu Arg Asp Glu Asp Asn Asn Leu Leu Thr Glu Lys Gln
   180 185 190
   Lys Leu Arg Val Lys Lys Ile His Glu Asn Glu Lys Arg Leu Glu Ala
   195 200 205
   Gly Asp His Pro Val Glu Leu Leu Ala Arg Asp Phe Glu Lys Asn Tyr
   210 215 220
   Asn Met Tyr Ile Phe Pro Val His Trp Gln Phe Gly Gln Leu Asp Gln
   225 230 235 240
   His Pro Ile Asp Gly Tyr Leu Ser His Thr Glu Leu Ala Pro Leu Arg
   245 250 255
   Ala Pro Leu Ile Pro Met Glu His Cys Thr Thr Arg Phe Phe Glu Thr
   260 265 270
   Cys Asp Leu Asp Asn Asp Lys Tyr Ile Ala Leu Asp Glu Trp Ala Gly
   275 280 285
   Cys Phe Gly Ile Lys Gln Lys Asp Ile Asp Lys Asp Leu Val Ile
   290 295 300
<210> 2
   <211> 22
   <212> RNA
   <213> Artificial
<220>
   <223> SPARC siRNA
<400> 2
   aacaagaccu ucgacucuuc cc 22
<210> 3
   <211> 18
   <212> DNA
   <213> Artificial
<220>
   <223> DNA Primer
<400> 3
   ccaaccgcga gaagatga 18
<210> 4
   <211> 20
   <212> DNA
   <213> Artificial
<220>
   <223> DNA Primer
<400> 4
   ccagaggcgt acagggatag 20
<210> 5
   <211> 19
   <212> DNA
   <213> Artificial
<220>
   <223> DNA Primer
<400> 5
   cagccgcttc acctacagc 19
<210> 6
   <211> 25
   <212> DNA
   <213> Artificial
<220>
   <223> DNA Primer
<400> 6
   ttttgtattc aatcactgtc ttgcc 25
<210> 7
   <211> 20
   <212> DNA
   <213> Artificial
<220>
   <223> DNA Primer
<400> 7
   gctcatcatc tggccatttt 20
<210> 8
   <211> 20
   <212> DNA
   <213> Artificial
<220>
   <223> DNA Primer
<400> 8
   accaacctac ggatgactcg 20
<210> 9
   <211> 19
   <212> DNA
   <213> Artificial
<220>
   <223> DNA Primer
<400> 9
   ccgaccgaat gcagaagga 19
<210> 10
   <211> 21
   <212> DNA
   <213> Artificial
<220>
   <223> DNA Primer
<400> 10
   acagagtatt tgcgctccga a 21
<210> 11
   <211> 21
   <212> RNA
   <213> Artificial
<220>
   <223> si-Scram
<400> 11
   gcucacagcu caauccuaau c 21

## Description

### FIELD OF THE INVENTION

The present invention relates to methods and compositions for the modulation of wound healing and/or the production of extracellular membrane components by modulating the activity and/or amount of secreted protein acidic and rich in cysteine (SPARC) protein. The invention further provides methods for identifying compounds useful in the above-mentioned methods and compositions.

### BACKGROUND OF THE INVENTION

Surgical intervention for many life threatening disorders involves the creation of deliberate wounds or the enlargement of existing channels to re-establish organ function and to alleviate the disease state. Examples include balloon angioplasty with stenting of narrowed coronary arteries and the formation of a fistula in glaucoma filtration surgery to maintain blood and aqueous humor flow, respectively.

Glaucoma is the most common cause of irreversible blindness worldwide. The World Health Organisation (WHO) estimates the number of people blinded from glaucoma in 2002 as 4.4 million (12.3% people in the world blind) (Resnikoff et al., Bull. World Health Organ. 2004, 82, 844-851). The number of people affected with glaucoma is predicted to be 79.6 million worldwide by 2020, of which over 8 million will suffer from bilateral blindness (Quigley and Broman, Br. J. Ophthalmol. 2006, 90, 151-156). Elevated intra ocular pressure (IOP) is the most important modifiable risk factor for the development and progression of glaucomatous optic neuropathy which can lead to eventual blindness (The AGIS investigators, Am. J. Ophthalmol. 2000, 130, 429-440). Surgery in the form of filtration surgery remains the most effective method of achieving a consistently desirable low IOP. Therein, a partial thickness flap is made in the scleral wall of the eye, and a window opening made under the flap to remove a portion of the trabecular meshwork. The scleral flap is then sutured loosely back in place. This allows fluid to flow out of the eye through this opening, resulting in lowered intraocular pressure and the formation of a bleb or fluid bubble on the surface of the eye. Scarring can occur around or over the flap opening, causing it to become less effective or completely lose effectiveness.

A major complication of glaucoma filtration surgery and angioplasty with stenting leading to an increased risk of surgical failure and morbidity is the post-operative wound healing response via obstruction of either the lumen or fistula by excessive extracellular matrix (ECM) remodeling (Lafont et aL, Circulation 1999, 100, 1109-1115; Reddick et al., Ann. Ophthalmol. 1985, 17, 200-203). In particular, in glaucoma filtration surgery the post-operative subconjunctival scarring response remains the final barrier to achieving long-term bleb function and surgical success.

In general, to limit the post-operative scarring response, anti-proliferative agents are often employed to improve surgical outcomes. The use of sirolimus in cardiac stenting (Morice et al., N. Engl. J. Med. 2002, 346, 1773-1780) and mitomycin C (MMC) and 5-fluorouracil (5FU) in glaucoma filtration surgery (The Fluorouracil Filtering Surgery Study Group, Am. J. Ophthalmol. 1989, 108, 625-635; Kitazawa et al., Arch. Ophthalmol. 1991, 109, 1693-1698; Skuta et al., Ophthalmol. 1992, 99, 438-444) are currently employed to improve surgical outcomes. In the eye, MMC and 5FU are titrated against risk factors for excess scar formation but despite their use, a significant failure rate persists and is associated with sight-threatening infections (Higginbotham et al., Ophthalmol. 1996, 103, 650-656; Greenfield et al., Arch. Ophthalmol. 1996, 114, 943-949). Therefore, the quest to find an alternative, safe, effective and targeted anti-fibrotic(s) is ongoing.

Due to the natural healing mechanisms, maintaining a functional wound requires deliberate intervention. Much is now understood of the normal wound healing mechanisms (Diegelmann & Evans, Front. Biosci. 2004, 9, 283-289). In normal wound healing, wound closure is accompanied by the eventual disappearance of granulation tissue consisting of fibroblasts, macrophages, blood vessels and ECM produced by fibroblasts. In pathological wound healing or fibrosis, however, excessive deposition of ECM, of which collagen is the most important, is known to cause aberrant tissue remodeling and the persistence of granulation tissue leading ultimately to pathological scarring. While collagen is needed to repair the wound defect and restore anatomic structure and function during normal wound healing, excessive collagen causes the lost of normal anatomical structure leading to compromised tissue function and fibrosis occurs (Lazarus et al., Arch. Dermatol. 1994, 130, 489-493). Hence, regulating ECM components, particularly collagen, is key to the outcome of the healing process.

SPARC (secreted protein acidic and rich in cysteine), also known as osteonectin and BM-40, is a 32 kDa calcium-binding matricellular protein involved in modulating cell-ECM interactions without contributing structurally to the ECM (Brekken and Sage, Matrix Biol. 2001, 19, 816-827; Bornstein and Sage, Curr. Opin. Cell Biol. 2002, 14, 608-616). Increased expression of SPARC has been observed in fibrotic disorders of the skin (Zhou et al., Arthritis Rheum. 2002, 46, 2990-2999; Zhou et al., Arthritis Rheum. 2006, 54, 2626-2631), lungs (Kuhn & Mason, Am. J. Pathol. 1995, 147, 1759-1769), kidneys (Pichler et al., Kidney Int. 1996, 50, 1978-1989; Kanauchi et al., Diabetologia 2000, 43, 1076-1077), liver (Frizell et al., Hepatol. 1995, 21, 847-854), and atherosclerotic vascular lesions (Dhore et al., Arterioscler. Thromb. Vasc. Biol. 2001, 21, 1998-2003). Conversely, the SPARC knockout (SPARC-/-) mouse exhibited diminished bleomycin-induced pulmonary fibrosis (Strandjord et al, Am. J. Physiol. 1999, 277, 628-635 in addition to other phenotypes all suggesting a compromised maturation and assembly of the ECM.

### SUMMARY OF THE INVENTION

The inventors have found here that SPARC expression and function play a critical role in wound healing, in particular in scar tissue formation.

Thus, in a first aspect, the present invention is directed to a pharmaceutical composition for use in the prevention of post operative wound scarring or preserving a surgically-induced functional wound after glaucoma filtration surgery, comprising reducing the activity and/or amount of SPARC protein (secreted protein acidic and rich in cysteine).

The SPARC protein may have the SWISS-PROT Databank entry No. P09486. In one embodiment, the SPARC protein comprises or consists of the amino acid sequence set forth in SEQ ID NO:1. The modulation may be in a cell or in a tissue and may be *in vivo* or *in vitro.*

In various embodiments the composition modulating the production of at least one extracellular matrix (ECM) component, comprising modulating the amount and/or the activity of SPARC protein (secreted protein acidic and rich in cysteine).

In various embodiments, the at least one extracellular matrix (ECM) component is an ECM protein, such as collagen I and/or fibronectin.

In one embodiment, the amount of SPARC protein may be reduced in tissue surrounding a wound site.

The amount and/or activity of SPARC protein may be reduced by reducing the expression of the nucleotide sequence encoding the SPARC protein. This reduction may include reducing the expression of the nucleotide sequence encoding for the SPARC protein. This may, for example, be achieved by means of a nucleic acid molecule, such as a non-coding nucleic acid molecule. The nucleic acid molecule may be RNA or DNA. In one specific embodiment of the invented methods, the nucleic acid molecule is selected from the group consisting of an DNA or RNA aptamer, an anti-sense-RNA molecule, a silencer-RNA molecule, a short hairpin RNA (shRNA) molecule, a micro RNA (miRNA) molecule, a small interfering RNA (siRNA) molecule and a repeat-associated small interfering RNA (rasiRNA) molecule. In one embodiment, the nucleic acid molecule is a small interfering RNA (siRNA) molecule, for example that comprising or consisting of the nucleotide sequence 5'-AACAAGACCUUCGACUCU UCCC-3' (SEQ ID NO:2).

The reduction of the amount and/or activity of SPARC may comprise the use of an agent that reduces the amount and/or activity of SPARC, the agent being selected from the group consisting of a peptide, a polypeptide, a peptoid, an inorganic molecule and a small organic molecule. In one embodiment, the agent is a polypeptide selected from the group consisting of an antibody, a fragment of an antibody, and a proteinaceous binding molecule with antibody-like functions. The peptide may be a peptide aptamer.

In various embodiments, the pharmaceutical composition for use in the prevention of post operative wound scarring or preserving a surgically-induced functional wound after glaucoma filtration surgery comprise reducing the amount and/or activity of SPARC protein in a subject in need thereof. This may include administering a therapeutically effective amount of a composition comprising an agent that reduces the amount and/or activity of a SPARC protein to the subject. Suitable agents are those indicated above. The subject may be an animal, preferably a mammal, such as a rat, a mouse, a rabbit, a guinea pig, an opossum, a squirrel, a nine-banded armadillo, a dog, a cat, an elephant, a chimpanzee, a rhesus monkey, a macaque, an orangutan, cattle (cow), a marmoset, an American pika, a galago or a human.

In further embodiments, the surgically-induced functional wound is a surgically placed fistula.

The invented may be used in a treatment for lowering intraocular pressure, such as, for example, glaucoma filtration surgery.

In various embodiments the pharmaceutical composition for reducing the amount and/or activity of SPARC protein (secreted protein acidic and rich in cysteine), comprises an agent that modulates the amount and/or activity of SPARC protein selected from the group consisting of a DNA or RNA aptamer, an anti-sense-RNA molecule, a silencer-RNA molecule, a short hairpin RNA (shRNA) molecule, a micro RNA (miRNA) molecule, a small interfering RNA (siRNA) molecule and a repeat-associated small interfering RNA (rasiRNA) molecule, a peptide, a peptoid, an inorganic molecule and a small organic molecule.

In one embodiment of the composition, the agent is siRNA. The siRNA may comprise or consist of the nucleotide sequence 5'-AACAAGACCUUCGACUCU UCCC-3' (SEQ ID NO:2).

The pharmaceutical composition may be in the form of liquid drops, an ointment, a gel, a therapeutic lens, a soluble ocular drug insert, and a collagen shield. The form may be suitable for topical administration.

In some embodiments, the composition is encapsulated in liposomes, polyelectrolyte capsules, or biodegradable polymeric carriers.

The pharmaceutical composition may further comprise one or more excipients, such as buffers, stabilizers, preservatives and the like.

In various embodiments, modulating the amount and/or activity of SPARC comprises modulating wound healing, such as inhibiting the formation of excess scar tissue and/or fibrosis in an animal. In one embodiment, this includes inhibiting ocular wound scarring.

### BRIEEF DESCRIPTION OF THE DRAWNINGS

In the following description, various embodiments of the invention are described with reference to the following Figures.

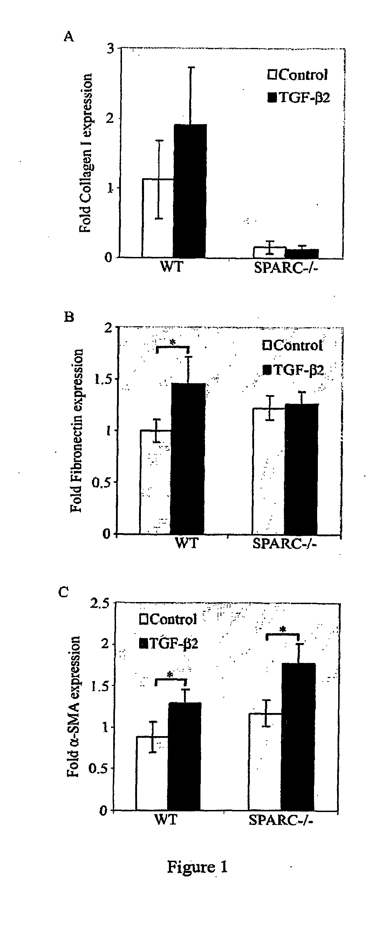

**Figure 1** shows the effect of TGF-β2 on a) collagen I, b) Fibronectin, and c) α-SMA expression in WT and SPARC-/- cells. (A) TGF-β2 upregulated collagen I mRNA expression in WT but not SPARC-/- conjunctival fibroblasts. The unstimulated level of collagen I mRNA in SPARC-/- cells is significantly lower than in WT control cells (*P*<0.05). (B) TGF-β2 up-regulated Fibronectin mRNA expression in WT (*, *P*<0.005) but not SPARC-/- conjunctival fibroblasts. (C) TGF-β2 up-regulated α-SMA mRNA expression in WT (*, *P*<0.005) and SPARC-/- (*, *P*<0.005) conjunctival fibroblasts. The housekeeping gene *β-actin* transcript was used for normalization. Data is shown as fold induction compared with unstimulated control WT cells. Data shown is representative of three independent experiments.

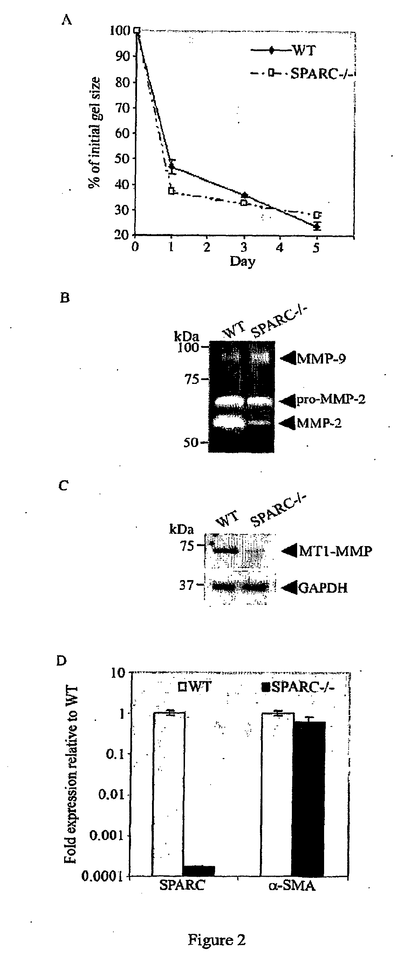

**Figure 2** shows that the reduction in the rate of collagen lattice contraction mediated by SPARC-/- fibroblasts is associated with reduced MT1-MMP expression and MMP-2 activity. (A) SPARC-/- conjunctival fibroblasts mediated less rapid free-floating collagen gel contraction in the extended period after 24 h compared to WT. WT or SPARC-/- fibroblasts were seeded in collagen solution in triplicate and allowed to polymerize. Gels were immediately detached and gel contraction was digitally photodocumented at day-1, -3 and -5. Gel contraction was measured as a reduction of gel surface area, expressed as a % of the initial gel size measured immediately after detachment. Values are the means of triplicates; bars, SD. The data shown is representative of three independent experiments. The rates of contraction from day-3 to day-5 are shown in Table I. (B) MMP-2 activity is reduced in medium conditioned by SPARC-/- fibroblasts seeded in contracting collagen gels. MMP-2 activity in medium conditioned by WT or SPARC-/- fibroblasts seeded in collagen lattices for 5 days was analysed by gelatin zymography. Proteolytic activity corresponding to the molecular weights of MMP-9 (92 kDa) and pro- and active MMP-2 (72 and 66 kDa respectively) are indicated by arrowheads. While activities for MMP-9 and pro-MMP-2 did not appear to be obviously different between WT and SPARC-/- conditioned media, MMP-2 activity was conspicuously reduced in the latter. (C) MT1-MMP expression is reduced in SPARC-/- fibroblasts. WT or SPARC/- fibroblasts seeded in collagen lattices for 5 days were lysed and 40 µg of each protein preparation subjected to Western analyses with antibodies specific for MT1-MMP and GAPDH as control. (D) The reduced rate of collagen gel contraction mediated by SPARC deficiency did not involve α-SMA. WT or SPARC/- fibroblasts seeded in collagen lattices for 5 days were lysed and the mRNA expressions of SPARC and α-SMA analysed by real-time quantitative PCR. Values shown are the means of triplicates; bar, SD.

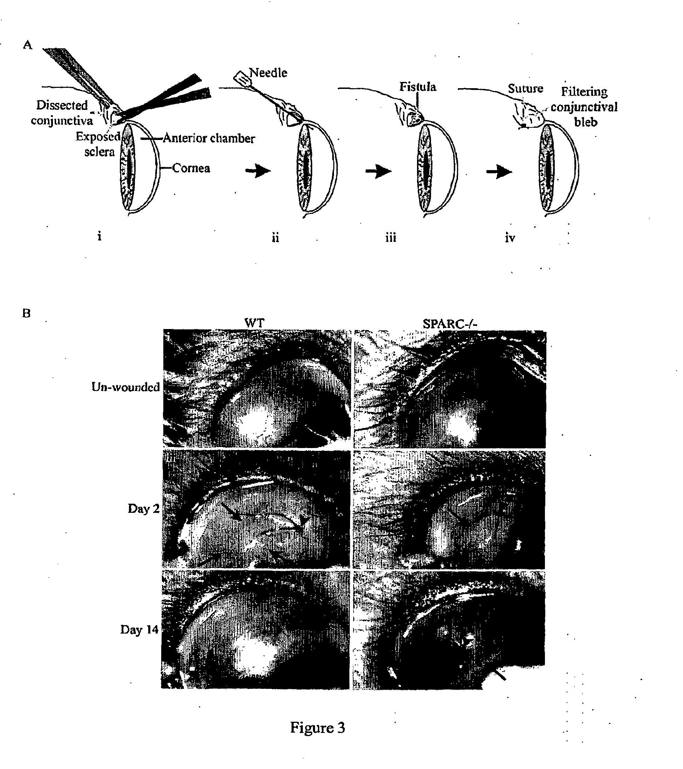

**Figure 3** shows the creation of a filtering wound in a mouse model of human glaucoma filtration surgery. (A) Schematic diagram of the surgical process involved in the creation of a functioning wound in the mouse conjunctiva. The conjunctiva was first surgically dissected to expose the underlying sclera (i) which was then cannulated with a 31.5 G cannula through the sclera into the anterior chamber of the eye (ii) so as to allow aqueous humor to flow out (red arrows, iii). Finally, the conjunctiva was closed by suturing over the newly created fistula. The conjunctiva overlying the wound site could be observed as a filtering bleb (iv). (B) Slit lamp examination of wound sites revealed the presence of the bleb in SPARC-/- conjunctiva 14-days post-surgery. The conjunctiva in the unwounded conjunctiva in contrast is flat and smooth (i, ii). On day-2 post-wounding, the wound site close to the suture (arrowhead) took on the appearance of a bleb (demarcated by arrows), suggesting functionality as aqueous fluid collected in the conjunctiva (iii, iv). On day-14 post-surgery, the WT bleb belonging to the same eye as (iii) has completely flattened down to superficially resemble the unwounded conjunctiva (v) while the SPARC-/- bleb belonging to the same eye in (iv) remained visibly elevated (vi, outlined by dotted line and demarcated by arrows).

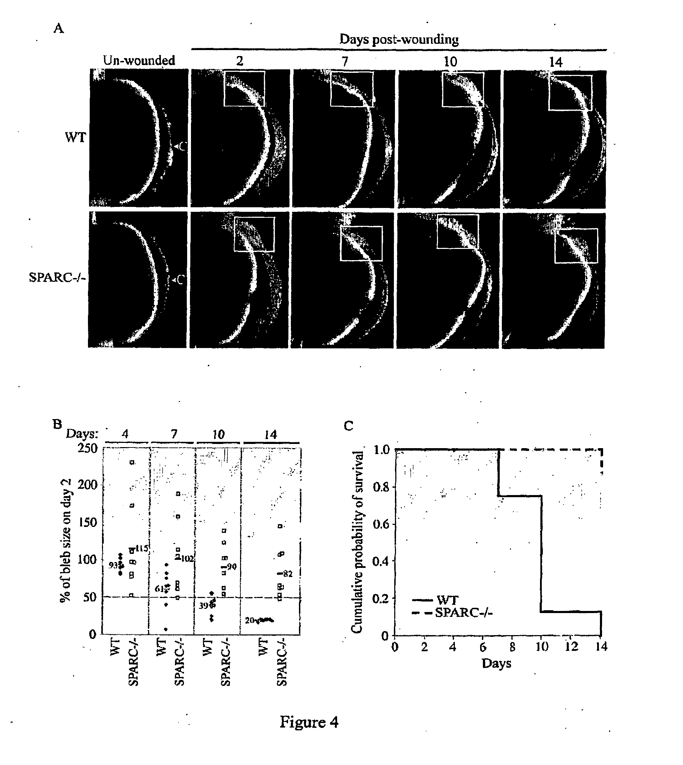

**Figure 4** shows that SPARC deficiency prolongs bleb survival. (A) Optical coherence tomography imaging of the mouse conjunctiva confirms bleb survival in the SPARC-/- conjunctiva. The unwounded and wounded eyes on day-2, -7, -10 and -14, were examined. The location of the bleb is indicated by the white box. The bleb is seen to progressively diminish in the WT wounded eye until its virtual disappearance on day-14, whereas the bleb remains visibly present in the SPARC-/- eye. Images shown belong to the same WT or SPARC-/- eye at each of the days examined and are representative of eight eyes of each genotype. C, cornea. (B) SPARC-/- bleb sizes are larger than WT. Each bleb at the indicated days post-wounding was measured centrally in at least 5 OCT imaging fields and the mean size expressed as a percentage of the initial size on day-2 post-surgery. Each symbol represents a single eye (◆, WT, n = 8; □, SPARC-/-, n=8). The horizontal bars and numbers indicate the mean % bleb sizes. (C) Keplan-Meier curve showing that targeted deletion of SPARC resulted in increased bleb survival compared to WT. Individual eyes were scored as positive for bleb survival when the bleb size is ≥50% of the initial day-2 bleb as shown in (B). Bleb failure in WT eyes progressed rapidly from 25% (2/8) on day-7 to 87.5% (7/8) and 100% (8/8) on days-10 and -14 post-surgery respectively while SPARC-/- blebs experienced only 12.5% failure (1/8) on day-14 post-surgery.

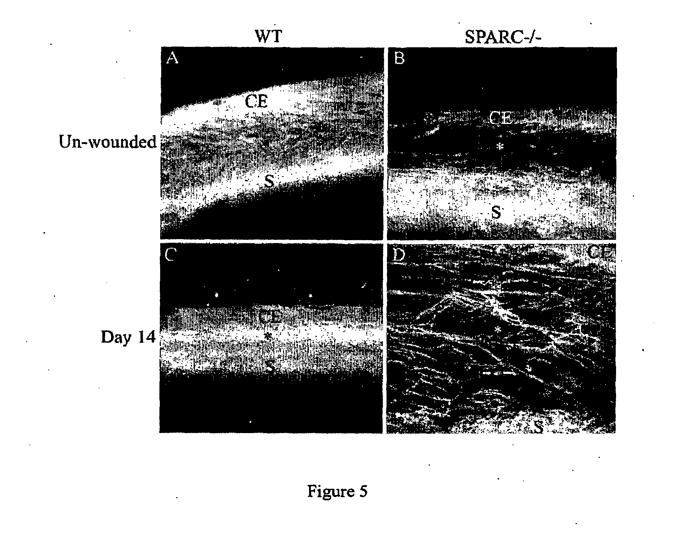

**Figure 5** shows that the differential organization of the SPARC-/- subconjunctival tissue matrix from WT is associated with a distinct wounding response. *In vivo* confocal imaging of the unoperated WT (A) and SPARC-/- (B) conjunctiva revealed an optical difference in the composition and organization of the subconjunctival matrix. On day-14 post-surgery, the WT subconjunctival matrix appeared optically dense and filled with a tightly interlaced fibrous material (C) while the SPARC-/- matrix was expanded and optically diffuse with a loosely organized fibrous network. Images shown are representative of at least 3 eyes of each genotype. CE, conjunctival epithelium; *, subconjunctival space; S, sclera.

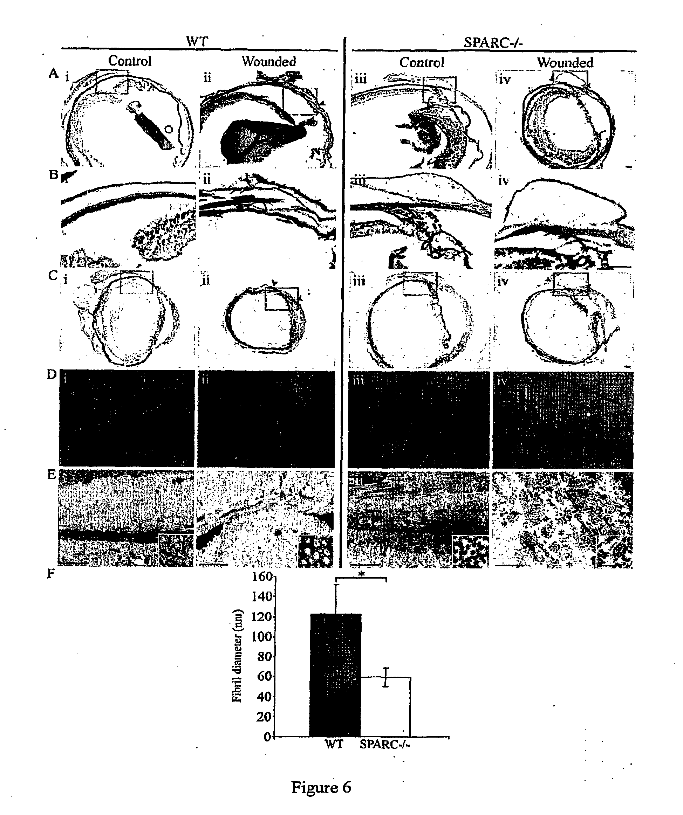

**Figure 6** shows that collagen deposition is altered in the SPARC-/- conjunctiva. (A) Hematoxylin and eosin staining revealed a flattened conjunctiva at the 14 day-old WT wound site (ii, arrowheads) while the SPARC-/- wound site was obvious as a spongy protuberance in the conjunctiva (iv, arrowheads). (B) Higher magnification of insets (boxed areas in A) illustrate differing thickness in the SPARC-/- (iii and iv) versus WT (I and ii) conjunctiva epithelium independent of wounding. (C) Picrosirius red-stained sections of the same eyes at low magnification and visualized by brightfield microscopy. (D) Higher magnification of insets (boxed areas in C) show collagenous matrix components more clearly which were identified by polarization microscopy as a meshwork organization of collagen I fibers in normal WT conjunctiva (i) compared to the well aligned and tightly packed fibers in WT wound (ii), and delicate meshwork in both control SPARC-/- conjunctiva (iii) and SPARC-/- bleb (iv). (E) Transmission electron microscopy confirmed a tightly packed matrix in the WT conjunctival wound (ii) in contrast to the presence of areas of loosely organized matrix with gaping holes in the SPARC-/- wound (*, iv). Ultrastructural analysis also illustrated the intrinsic deposition of smaller collagen fibrils in the unoperated SPARC-/- eye (iii, inset) compared to WT (i, inset). (F) Graphical representation of the mean diameter of collagen fibrils (± SD) from about 10 fields of view of unoperated WT (filled bar, n = 400) and SPARC-/- conjunctiva (open bar, n = 405). *, *P*<0.001. Bars: (A to D) 100 µm; (E) 1 µm; (E, insets) 200 nm.

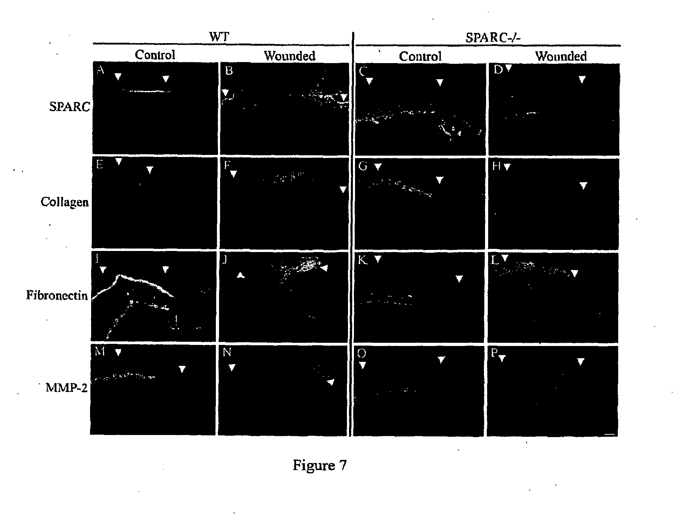

**Figure 7** shows that SPARC-/- conjunctive wound features reduced levels of ECM proteins. Immunofluorescence analysis of normal conjunctiva and wound sites in the WT and SPARC-/- eye revealed that wounding in the WT conjunctiva (arrowheads) upregulated the expression of SPARC (B), collagen I (F), fibronectin (J) and MMP-2 (N) compared to normal WT conjunctiva (arrowheads, A, E, I, M). The SPARC-/- conjunctiva (arrowheads) demonstrated no SPARC expression (C, D) and low level expressions of collagen I (G), and MMP-2 (O) which were not upregulated upon surgical wounding (arrowheads, H, P). There was some upregulation in fibronectin expression in the SPARC-/- bleb (L) but this appeared more diffused in comparison to the WT surgical wound site (J). The mouse anti-SPARC and anti-fibronectin antibodies were visualized with anti-mouse secondary antibody conjugated with Alexa Fluro 488 while rabbit anti-collagen and anti-MMP-2 were visualized with anti-rabbit secondary antibody conjugated with Alexa Fluro 594. Bar, 100 µm.

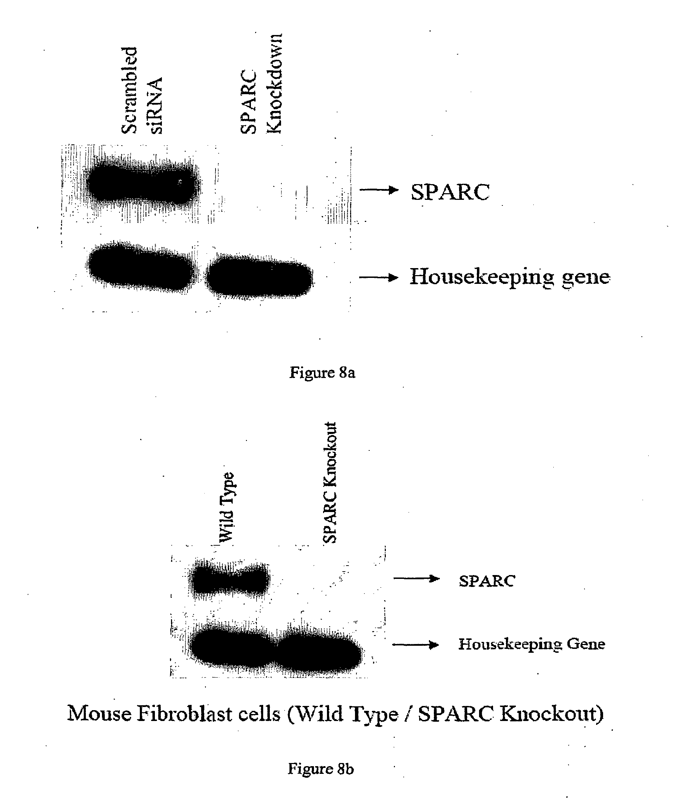

**Figure 8a** shows a Western Blot of si-SCRAM and si-SPARC transfected human tenon's fibroblasts protein lysate. Human tenon's fibroblasts were transfected with 100 mM either scrambled siRNA (designated si-Scram) or siRNA targeting SPARC (designated si-SPARC) for 7 days and the expression of SPARC was examined by Western blotting with anti-SPARC and anti-β-tubulin (loading control) antibodies. A significant knockdown of the SPARC gene is evident and is comparable to the absence of SPARC observed in Figure 8b, illustrating the absence of SPARC protein in a genetic knockout mouse.
**Figure 8b** shows a Western Blot of Wild Type and SPARC knockout mouse fibroblast cells protein lysate. In the knockout mouse fibroblast cells no SPARC protein can be detected. Hence, the *sparc* gene is successfully knocked out in the mouse cells.

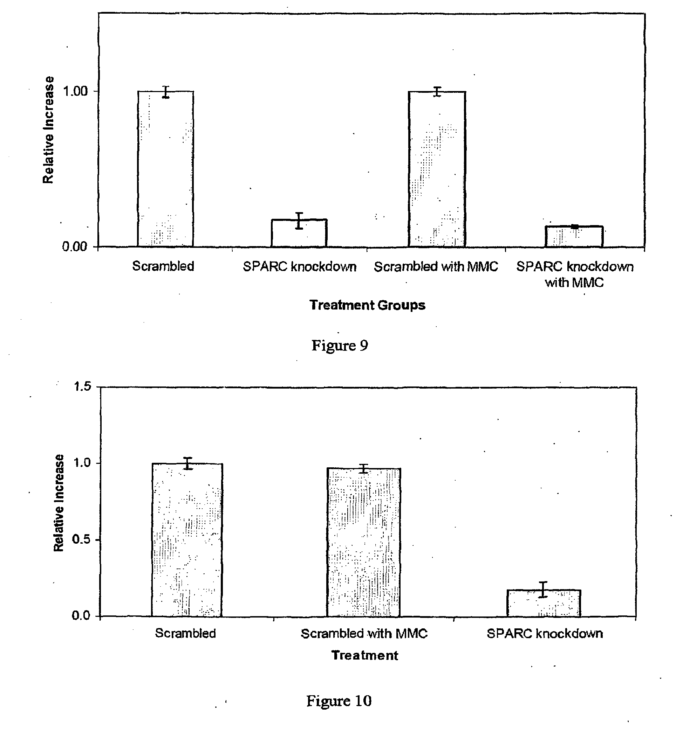

**Figure 9** shows the RNA expression of SPARC in SPARC knockdown cells in collagen gels with or without treatment with MMC.
**Figure 10** shows the RNA expression of SPARC in Scrambled siRNA and SPARC siRNA transfected cells with the treatment of MMC in collagen gels (day 0).

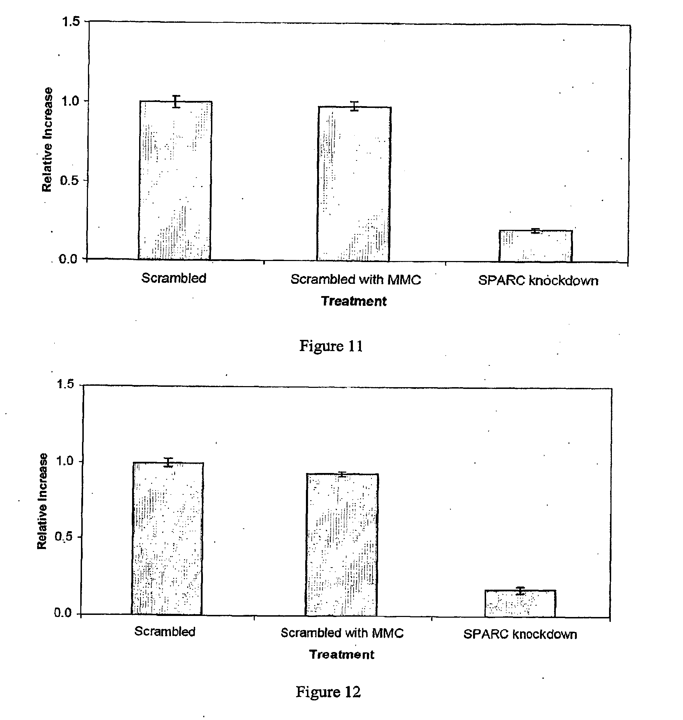

**Figure 11** shows the RNA expression of SPARC in Scrambled siRNA and SPARC siRNA transfected cells with the treatment of MMC in collagen gels (day 3).
**Figure 12** shows the RNA expression of SPARC in Scrambled siRNA and SPARC siRNA transfected cells with the treatment of MMC in collagen gels (day 7).

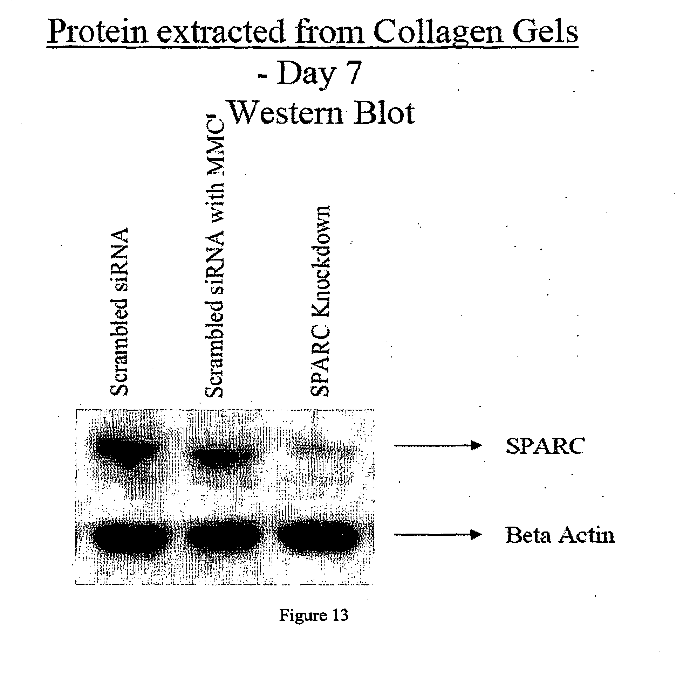

**Figure 13** shows a Western Blot of proteins extracted from collagen gels after day 7. Human fibroblast cells were *in vitro* transfected with either scrambled si-RNA or si-SPARC and exposed to a collagen gel for 7 days. Proteins were isolated from the gel and analyzed with a Western Blot analysis for SPARC and β-Actin expression.

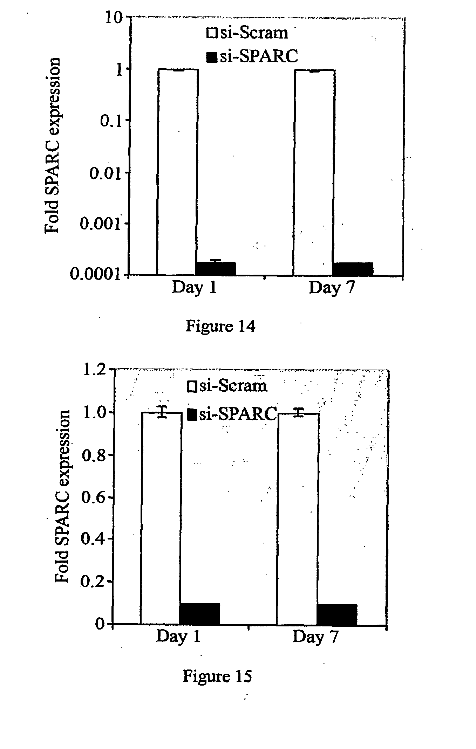

**Figure 14** shows the relative SPARC expression on day 1 and day 7 of si-SPARC and si-SCRAM transfected human tenon's fibroblasts. Human tenon's fibroblasts were transfected with either scrambled siRNA (designated si-Scram) or siRNA targeting SPARC (designated si-SPARC) for the indicated number of days and the expression of SPARC was examined by quantitative real time-PCR.
**Figure 15** shows the relative SPARC expression on day 1 and day 7 of si-SPARC and si-SCRAM transfected mouse conjunctive fibroblasts. Mouse fibroblasts were transfected with either scrambled siRNA (designated si-Scram) or siRNA targeting SPARC (designated si-SPARC) for the indicated number of days and the expression of SPARC was examined by quantitative real time-PCR.

### DETAILED DESCRIPTION

The present invention relates to methods and compositions for the modulation of wound healing and/or the production of extracellular membrane components by modulating the activity and/or amount of secreted protein acidic and rich in cysteine (SPARC) protein. The invention further provides methods for identifying compounds useful in the above-mentioned methods and compositions.

The word "exemplary" is used herein to mean "serving as an example, instance, or illustration". Any embodiment or design described herein as "exemplary" is not necessarily to be construed as preferred or advantageous over other embodiments or designs.

In the context of the various embodiments, the terms used have the meaning commonly accepted in the art unless explicitly indicated otherwise

The inventors of the present invention have surprisingly found that by modulating the intracellular level of SPARC protein, ECM expression - in particular collagen expression - can be modulated. Specifically, it has been found that the expression of collagen is reduced in response to the downregulation of SPARC expression. Furthermore, it has surprisingly been found that SPARC-/- conjunctival fibroblasts fail to increase collagen I or fibronectin expression upon addition of exogenous TGF-β2 *in vitro,* which suggests a central involvement of SPARC in the TGF-β signaling pathway. The canonical TGF-β signaling pathway involving Smads (Shi and Massagué, Cell 2003, 113, 685-700) is known to be involved in regulating collagen and fibronectin transcription (Ramirez et al., Matrix Biol. 2006, 25, 365-372; Itoh et al., J. Biol. Chem. 2003, 278, 3751-3761). SPARC is probably not involved in regulating the availability of TGF-β to its receptors as was suggested previously (Schiemann et al., Mol. Biol. Cell 2003, 14, 3977-3988) since that would imply a curtailment of TGF-β signaling in SPARC-/- cells. Instead, it was found out that the growth factor it still able to stimulate α-SMA expression in *in vitro* studies. Consequently, myofibroblast formation, as represented by α-SMA expression, is not affected in response to TGF-β induction in SPARC-depleted conjunctival fibroblasts. Without wishing to be bound to this hypothesis, this implies that the formation of contractile granulation tissue is not affected in a SPARC-deficient wound. However, this granulation tissue is not functioning as efficiently as WT cells, as the inventors demonstrated that SPARC-/-cells mediated slower three-dimensional collagen gel contraction compared to WT cells.

The slower pace in contraction may be attributed to a decrease in MMP-2 activity which may in turn be due to the down-regulation of MT1-MMP expression. In support, SPARC has previously been shown to regulate MT1-MMP activity (McClung et al., Neurosci. Lett. 2007, 419, 172-177). Both MMP-2 activity (Seltzer et al., Exp. Cell. Res. 1994, 213, 365-374; Deryugina et al., Cancer Res. 1998, 58, 3743-3750) and up-regulation of MT1-MMP (Tomasek et al., J. Biol. Chem. 1997, 272, 7482-7487), which cleaves and activates MMP-2 (Sato et al., Nature 1994, 370, 61-65), are known to play critical roles in mediating collagen gel contraction via degradation of the ECM to facilitate the migration of cells and remodel the new ECM. A reduction in MMP-2 activity is likely to decrease matrix remodelling and cause the observed reduced rate of collagen matrix contraction. Since contraction speeds wound closure, the slower contraction rate in the absence of SPARC aids in the delay of wound closure. Moreover, MMP-2 is known to regulate cell migration and angiogenesis, both of which can contribute positively to fibrosis (Wynn, J., Clin. Invest. 2007, 117, 524-529). Hence, the reduced MMP-2 expression in the SPARC-/- wound is an important factor in maintaining wound functionality by delaying wound contraction as well as inhibiting fibrotic wound healing.

Using a murine model of glaucoma filtration surgery, a novel therapeutic indication for SPARC inhibition in maintaining a surgically-induced functional wound through favourable post-operative changes in the organization of the ECM has been found. SPARC expression can be suppressed in both human and mouse subconjunctiva by the application of siRNA. siRNA targeting SPARC or other means to modulate SPARC therefore can form the critical constituent of a therapeutic anti-scarring formulation to be delivered during glaucoma filtration surgery to maintain and prolong bleb function and so help the glaucoma patient requiring such a surgery to attain and maintain a normal intraocular eye pressure post-surgery.

Employing the *in vivo* model described herein, it was found that WT surgeries failed as a result of aqueous outflow obstruction by excessive deposition of collagen I over the fistula. Moreover, the densely packed collagen I was organized in a mechanically inefficient manner thus preventing aqueous filtration, and further contributing to bleb failure. Hence, these findings support the notion that bleb failure in the WT mice is a result of a massive induction and disorganized deposition of ECM. In contrast thereto, in SPARC-/- animals the wound remain "open" and filtering, and was associated with reduced collagen I fibers that were assembled as a loose stromal network in the subconjunctival space. The smaller collagen fibril diameters in the SPARC-/- conjunctiva positively contribute to the concept of the "open" wound phenotype. Thus, SPARC deficiency can maintain a functional wound that has been surgically created in the eye. Specifically, the inventors found out in an *in vivo* model that such a wound remains advantageously functional in 87.5% of the SPARC deficient mice whereas none of the wounds in WT mice were functional at 14 days post-surgery. The extended functionality of the wound is due to a favourable ECM composition of SPARC modulated wounds, since less collagen I is detected compared to the control conditions.

Thus, the methods and compositions described herein are based on the identification of SPARC depletion as an effective therapeutic target in preserving surgically-induced functional wounds.

Hence, in a first aspect the invention relates to a method for the modulation of wound healing by modulating the amount and/or activity of SPARC protein. The modulation of the amount and/or activity of SPARC protein may be achieved by modulating the expression of the nucleic acid encoding the SPARC protein.

In a second aspect, the invention relates to a method for the modulation of the production of at least one extracellular matrix (ECM) component by modulating the amount and/or activity of SPARC protein. Again, the modulation of the amount and/or activity of SPARC protein may be achieved by modulating the expression of the nucleic acid encoding the SPARC protein.

As already detailed above, SPARC (secreted protein acidic and rich in cysteine), also known as osteonectin and BM-40 (SWISS-PROT Databank entry No. P09486;), is a 32 kDa calcium-binding matricellular protein involved in modulating cell-ECM interactions without contributing structurally to the ECM. The SPARC protein may comprise or consist of the amino acid sequence set forth in SEQ ID NO:1.

"Wound healing" as used herein refers to the healing of a wound including the formation of new, specialized tissue and restoration of function, in particular the closure of a wound. Healing of a wound thus includes the proliferation of cells and the formation of new tissue, including scar tissue. Consequently, the modulation of wound healing also includes the prevention of wound healing, the modulation of new tissue formation, in particular scar tissue formation, preferably the reduction of wound scarring by inhibiting the formation of scar tissue. Also included is the modulation, preferably the reduction or inhibition of fibrotic growth and fibrosis. In various embodiments of the invented methods, the wound may be a post operative wound and the method may be used to prevent post operative scarring or fibrosis. Particularly preferred is the use of the method after ocular surgery, especially glaucoma filtration surgery, to prevent ocular scarring. Accordingly, the method may be used to lower intraocular pressure. In certain embodiments, the method may be used for preserving a surgically-induced functional wound, such as a surgically placed fistula.

"Modulation" or "modulating" as used herein includes reduction as well as the increase of the corresponding variable. For example, "modulating the amount" thus includes increases in the amount as well as the reduction of the amount.

Generally, the modulation may be in a cell or in a tissue and may be *in vivo* or *in vitro.* Thus, the method may be performed on cells or tissue in cell or tissue culture or may be performed on a living being.

Modulating the amount of the SPARC protein may include allowing a portion of the total number of the SPARC protein molecules, present in a cell, in an *in vitro* set-up such as a test tube or in an organism (*in vivo),* to be degraded. Degradation of the SPARC protein may for instance be achieved by use of a protease, including but not limited to calpain protease (also termed calcium-activated protease or calpain). Modulating the amount of the SPARC protein may also include modulating the expression of the SPARC protein. This may for example be carried out by administering a compound that modulates the expression of the SPARC protein. The expression of the SPARC protein may be increased or decreased (reduced). One example may be the use of a compound which acts on the SPARC operon and thereby influences the transcription of the SPARC mRNA, i.e., reduces the mRNA transcription. Due to a reduced mRNA transcription, the SPARC protein level is also reduced.

An operon is a functional entity of genomic material containing a cluster of units. The operon comprises a promoter, which is a nucleotide sequence that enables a gene to be transcribed. The promoter is recognized by RNA polymerase, which then initiates transcription. In RNA synthesis, the promoter indicates which genes should be used for messenger RNA creation and, by extension, control which proteins the cell manufactures. The operon further comprises an operator, a segment of the nuclei acid sequence to which a repressor can bind to. In the case a repressor binds to the operator, the repressor prevents the RNA polymerase from transcribing the structural gene(s) of the operon. Moreover, the operon comprises at least one structural gene, which encodes for a protein which's mRNA transcription is regulated by the operon.

Accordingly, for the reduction of the protein level a compound may be used, which binds to the SPARC operon, e.g. to the promoter region, and thereby prevents binding of the RNA polymerase to the operon, e.g. the promoter, or prevents the completion of the transcription. In the end either the transcription is abolished or incomplete. Alternatively, another compound may be used, which binds to a SPARC repressor or the SPARC operator and thereby induces/enhances repressor binding to the operator and reduces the SPARC mRNA translation.

As an alternative example, the expression of the SPARC protein may be reduced by means of a nucleic acid molecule, such as a non-coding nucleic acid molecule.

The term "nucleic acid molecule" as used herein refers to any nucleic acid in any possible configuration, such as single stranded, double stranded or a combination thereof. Nucleic acids include for instance DNA molecules (e.g., cDNA or genomic DNA), RNA molecules (e.g., mRNA), analogues of the DNA or RNA generated using nucleotide analogues or using nucleic acid chemistry, and PNA (protein nucleic acids). In various embodiments of the invention, the nucleic acid utilized for modulating SPARC activity and/or amounts is DNA or RNA. The DNA or RNA may be of genomic or synthetic origin and may be single or double stranded. Such nucleic acid can be e.g. mRNA, cRNA, synthetic RNA, genomic DNA, cDNA, synthetic DNA, a copolymer of DNA and RNA, oligonucleotides, etc. A respective nucleic acid may furthermore contain non-natural nucleotide analogues and/or be linked to an affinity tag or a label. In some embodiments the nucleic acid molecule may be isolated, enriched, or purified. The nucleic acid molecule may for instance be isolated from a natural source by cDNA cloning or by subtractive hybridization. The natural source may be mammalian, such as human, and includes bodily fluids, such as blood or semen, or tissue. The nucleic acid may also be synthesized, e.g. by the triester method or by using an automated DNA synthesizer.

Affinity tags to be used herein are known in the art and include His-Tags, FLAG-Tags and Myc-Tags. Similarly, suitable labels are also known and include, but are not limited to radio labels, fluorescent and chemiluminescent labels, including dyes and/or proteins, enzymes, epitopes and haptens.

Many nucleotide analogues are known and can be used in nucleic acids and oligonucleotides used in the present method of the invention. A nucleotide analogue is a nucleotide containing a modification at for instance the base, sugar, or phosphate moieties. Modifications at the base moiety include natural and synthetic modifications of A, C, G, and T/U, different purine or pyrimidine bases, such as uracil-5-yl, hypoxanthin-9-yl, and 2-aminoadenin-9-yl, as well as non-purine or non-pyrimidine nucleotide bases. Other nucleotide analogues serve as universal bases. Universal bases include 3-nitropyrrole and 5-nitroindole. Universal bases are able to form a base pair with any other base. Base modifications often can be combined with for example a sugar modification, such as for instance 2'-O-methoxyethyl, e.g. to achieve unique properties such as increased duplex stability.

In some embodiments of the present method of the invention the nucleic acid molecule is a non-coding nucleic acid molecule, such as for example an aptamer or a Spiegelmer^{®} (described in WO 01/92655). A non-coding nucleic acid molecule may also be an nc-RNA molecule (see e.g. Costa, FF, Gene (2005), 357, 83-94 for an introduction on natural nc-RNA molecules). Examples of nc-RNA molecules include, but are not limited to, an anti-sense-RNA molecule, an L-RNA Spiegelmer^{®}, a silencer-RNA molecule (such as the double-stranded Neuron Restrictive Silencer Element), a micro RNA (miRNA) molecule, a short hairpin RNA (shRNA) molecule, a small interfering RNA (siRNA) molecule, a repeat-associated small interfering RNA (rasiRNA) molecule or an RNA that interacts with Piwi proteins (piRNA) (for a brief review see e.g. Lin, H., Science (2007) 316, 397). An illustrative example of a suitable siRNA for the reduction of SPARC expression is 5'-AACAAGACCUUCGACUCU UCCC-3'.

The use of small interfering RNAs has become a tool to "knock down" specific genes. It makes use of gene silencing or gene suppression through RNA interference (RNAi), which occurs at the posttranscriptional level and involves mRNA degradation. RNA interference represents a cellular mechanism that protects the genome. siRNA molecules mediate the degradation of their complementary RNA by association of the siRNA with a multiple enzyme complex to form what is called the RNA-induced silencing Complex (RISC). The siRNA becomes part of RISC and is targeted to the complementary RNA species which is then cleaved. This leads to the loss of expression of the respective gene (for a brief overview see Zamore, PD, & Haley, B (2005) Science 309, 1519-1524). This technique has for example been applied to silencing parasitic DNA sequences, such as the cleavage of HIV RNA, as disclosed in US patent application 2005/0191618.

A typical embodiment of such a siRNA for the current invention includes an *in vitro* or *in vivo* synthesized molecule of 10 to 35 nucleotides, in some embodiments 15 to 25 nucleotides. A respective siRNA molecule may be directly synthesized within a cell of interest (including a cell that is part of a microorganism and an animal). It may also be introduced into a respective cell and/or delivered thereto. An illustrative example of delivering a siRNA molecule into selected cells *in vivo* is its non-covalent binding to a fusion protein of a heavy-chain antibody fragment (Fab) and the nucleic acid binding protein protamin (Song, E. et al. (2005), Nature Biotech. 23, 6, 709-717). The internalization of the Fab fragment shuttles the nucleic acid sequence into the cell. In further embodiments, the siRNA is delivered into the target cell using liposomes, transfection agents, polyelectrolyte capsules, biodegradable polymeric carrier capsules, nanoparticles, quantum dots, viruses, cell membrane pore-forming agents or protein transduction domains, such as protein transduction domains from pathogens, such as Clostridia or Anthrax. Alternatively, the nucleic acid may be internalized by endocytosis, electroporation or further means.

In an embodiment of the present invention, siRNA molecules are used to induce a degradation of mRNA molecules encoding one or more transcription factors involved in the (e.g. *in vivo*) synthesis of the SPARC protein.

Some embodiments of the method according to the invention include modulating the activity of SPARC protein. Modulating the activity of the SPARC protein may include forming a complex between the SPARC protein and a compound. This may for example be carried out by administering a compound that modulates the activity of the SPARC protein. The activity of the SPARC protein may be increased or decreased (reduced).

In a further embodiment, the function of SPARC may be hampered by the administration of SPARC fragments, which hinder the endogenous SPARC from exhibiting its activity, for example by competitive binding to SPARC binding sites without having the activity of the full length SPARC protein.

Modulating the activity of a SPARC protein may also include altering a posttranslational modification of the SPARC protein, including altering the pattern of posttranslational modifications of the SPARC protein. In some embodiments altering a posttranslational modification of the SPARC protein includes the use of a compound that alters the pattern of posttranslational modifications of the SPARC protein. Illustrative example of a respective posttranslational modification are phosphorylation, dephosphorylation, acetylation, glycosylation, deglycosylation, sulfatation and ubiquitylation. As a result of a respective posttranslational modification, e.g. phosphorylation, the activity of the SPARC protein can be changed.

A compound that modulates the activity and/or amount of a SPARC protein, for example by modulating the expression of a nucleic acid molecule that encodes the SPARC protein, may be any compound. Examples include, but are not limited to, a nucleic acid (see above), a peptide, a polypeptide, a peptoid, an inorganic molecule and a small organic molecule. Peptoids can have much higher cell permeability than peptides (see e.g. Kwon, Y.-U., and Kodadek T., (2007), J. Am. Chem. Soc. 129, 1508-1509). A peptide may be of synthetic origin or isolated from a natural source by methods well-known in the art. The natural source may be mammalian, such as human, blood, semen, or tissue. A peptide or polypeptide may for instance be synthesized using an automated polypeptide synthesizer. Illustrative examples of polypeptides are an antibody, a fragment thereof and a proteinaceous binding molecule with antibody-like functions. Examples of (recombinant) antibody fragments are Fab fragments, Fv fragments, single-chain Fv fragments (scFv), diabodies or domain antibodies (Holt, L.J., et al., (2003) Trends Biotechnol., 21, 11, 484-490). An example of a proteinaceous binding molecule with antibody-like functions is a mutein based on a polypeptide of the lipocalin family (WO 03/029462, Beste et al., (1999) Proc. Natl. Acad. Sci. U.S.A., 96, 1898-1903). Lipocalins, such as the bilin binding protein, the human neutrophil gelatinase-associated lipocalin, human Apolipoprotein D or glycodelin, posses natural ligand-binding sites that can be modified so that they bind to selected small protein regions known as haptens. Examples of other proteinaceous binding molecules are the so-called glubodies (see WO 96/23879), proteins based on the ankyrin scaffold (Mosavi, L.K., et al., (2004) Protein Science 13, 6, 1435-1448) or crystalline scaffold (WO 01/04144) the proteins described in Skerra, (2000) J. Mol. Recognit. 13, 167-187, AdNectins, tetranectins, and avimers. Avimers contain so called A-domains that occur as strings of multiple domains in several cell surface receptors (Silverman, J., et al., (2005) Nature Biotechnology 23, 1556-1561). Adnectins, derived from a domain of human fibronectin, contain three loops that can be engineered for immunoglobulin-like binding to targets (Gill, D.S. & Damle, N.K., (2006) Current Opinion in Biotechnology 17, 653-658). Tetranectins, derived from the respective human homotrimeric protein, likewise contain loop regions in a C-type lectin domain that can be engineered for desired binding (ibid.). Peptoids, which can act as protein ligands, are oligo(N-alkyl) glycines that differ from peptides in that the side chain is connected to the amide nitrogen rather than the α carbon atom. Peptoids are typically resistant to proteases and other modifying enzymes and can have a much higher cell permeability than peptides (see e.g. Kwon, Y.-U., and Kodadek, T., (2007) J. Am. Chem. Soc. 129, 1508-1509).Where desired, a modifying agent may be used that further increases the affinity of the respective moiety for any or a certain form, class etc. of target matter.

The peptides may be peptide aptamers. Such peptide aptamers consist of a short variable peptide domain, attached at both ends to a protein scaffold. This double structural constraint greatly increases the binding affinity of the peptide aptamer to levels comparable to an antibody's (nanomolar range). The variable loop length is typically composed of ten to twenty amino acids. Currently, the bacterial protein Thioredoxin-A is the most frequently used scaffold protein, the variable loop being inserted within the reducing active site, which is a -Cys-Gly-Pro-Cys- loop in the wild protein, the two cysteines lateral chains being able to form a disulfide bridge. Peptide aptamer selection can be made using different systems, but the most used is currently the yeast two-hybrid system.

In one embodiment, a proteinaceous binding molecule such as an antibody or a fragment thereof (or a lipocalin mutein also known as "Anticalin®, for example") binds to the SPARC protein and is therefore capable of neutralising its functional ability to organise the extracellular matrix and generate fibrosis and scarring in the conjunctiva following glaucoma surgery.

The small organic compounds have typically a molecular weight of up to 1000 g/mol and may be derived from or library of compounds used for drug screening. Suitable compounds may be found by *in silico* screening based on the structure of the SPARC protein. The compounds may for example block the SPARC binding site or, as allosteric effectors, bind to SPARC at regions distinct from the binding site and induce conformational changes that decrease SPARC binding to its natural binding partners.

The extracellular matrix component (ECM) component referred to above may comprise extracellular matrix proteins. Extracellular matrix proteins may be selected from the group comprising: collagen, collagen I, collagen II, collagen III, collagen IV, collagen V, collagen VI, collagen VII, collagen VIII, collagen IX, collagen X; collagen XI, collagen XII, fibronectin and laminin. Preferably, the ECM components are collagen I and/or fibronectin.

According to the above methods, the modulation of the amount and/or activity of the SPARC protein may include the reduction of the amount and/or activity of the SPARC protein. This modulation may be carried out locally, for example in the tissue surrounding the wound.

The method may be performed on a subject in need thereof In such an embodiment, modulating the amount and/or activity of SPARC protein can comprise administering a therapeutically effective amount of a composition comprising an agent that modulates the amount and/or activity of a SPARC protein to the subject. The subject may be an animal, preferably a mammal. The mammal may for example be selected from the group consisting of a rat, a mouse, a rabbit, a guinea pig, an opossum, a squirrel, a nine-banded armadillo, a dog, a cat, an elephant, a chimpanzee, a rhesus monkey, a macaque, an orangutan, a cattle (cow), a marmoset, an American pika, a galago and a human.

Also encompassed by the present invention is a method of screening for therapeutically effective compositions suitable for modulating the amount and/or activity of SPARC protein. The modulation may include modulating the expression of a nucleic acid molecule encoding SPARC protein and the modulation of wound healing. The method comprises setting a surgically-induced functional wound in a test animal; administering to the animal the test composition; and determining the effect of the composition on the wound healing in the animal. The determining step may include the comparison with wound healing in an animal that has not been administered the test composition (negative control) and/or an animal in which the SPARC gene has been knocked out or inactivated (positive control). A composition may be considered therapeutically active, if, compared to the negative control, wound healing, in particular scar tissue formation is slowed or abolished.

The reference animal in which the SPARC gene has been inactivated may be a SPARC-/- animal is used. This animal may be genetically engineered to have the SPARC genes turned off/to make them inoperable through a targeted mutation.

In various embodiments of this method, the surgically-induced functional wound is a surgically created fistula in the eye. The modulation of wound healing can comprise the inhibition of scar tissue formation, especially excess scar tissue formation, and/or fibrosis.

The test composition may comprise a nucleic molecule or derivative thereof that inhibits expression of SPARC protein. In one embodiment, the nucleic acid molecule is a siRNa molecule that targets the SPARC mRNA.

In the above methods, the test animal may be a mammal, optionally selected from the group consisting of a rat, a mouse, a rabbit, a Guinea pig, an opossum, a squirrel, a nine-banded armadillo, a dog, a cat, an elephant, a chimpanzee, a rhesus monkey, a macaque, an orangutan, a cattle (cow), a marmoset, an American pika, a galago, and a human. In preferred embodiments, the animal is a mouse, a rat or a rabbit.

These methods may be implemented in a murine model of experimental filtration surgery. This surgical model provides a valuable tool for evaluating post-operative wound healing with three important advantages: firstly, the eye is easily accessible to experimental surgery or application of therapeutics; secondly, the post-surgical outcome can be easily observed and readily quantified by a variety of available, well-established and independent imaging techniques which are all currently used in the clinical setting for patients so that in vivo data generated can be directly compared with that in humans; thirdly, the long-term surgical survival data can be evaluated over the lifetime of the animal if necessary.

The murine model has been validated in previous concepts of wound closure. This model effectively supports the belief that the primary mechanism of wound closure occurs via connective tissue matrix deposition whereby new ECM is formed with newly deposited collagen and ECM proteins (Diegelmann, J. Urol. 1997, 157, 298-302). Moreover, the surgical effect of the model reflected perfectly and predictably the most likely outcome of glaucoma filtration surgery in the absence of anti-proliferative agents. Indeed, bleb failure from wound obstruction has been mainly attributed to the accumulation of collagen (Esson et al., Invest. Ophthalmol. Vis. Sci. 2004, 45, 485-491), an effect that is elegantly demonstrated in this model. Thus, this surgical model is an invaluable tool for investigating potential therapeutics effective in inhibiting wound closure and/or maintaining a surgically created channel.

In a further aspect, the present invention relates to a pharmaceutical composition for modulating the amount of SPARC protein (secreted protein acidic and rich in cysteine) wherein the pharmaceutical composition comprises an agent that modulates the amount and/or activity of SPARC protein selected from the group consisting of a DNA or RNA aptamer, an anti-sense-RNA molecule, a silencer-RNA molecule, a short hairpin RNA (shRNA) molecule, a micro RNA (miRNA) molecule, a small interfering RNA (siRNA) molecule and a repeat-associated small interfering RNA (rasiRNA) molecule, a peptide, a peptoid, an inorganic molecule and a small organic molecule.

In one specific embodiment, the agent is siRNA, in particular a siRNA comprising or consisting of the nucleotide sequence 5'-AACAAGACCUUCGACUCU UCCC-3' (SEQ ID NO:2).

The pharmaceutical composition can be in the form of liquid drops, an ointment, a gel, a therapeutic lens, a soluble ocular drug insert, or a collagen shield. The composition may also be encapsulated in liposomes, polyelectrolyte capsules, or biodegradable polymeric carriers. Generally, the composition may comprise one or more excipients.

The pharmaceutical compositions of the invention may be used for modulating the amount and/or activity of SPARC. This use may comprise modulating wound healing. Again, this may comprise inhibiting the formation of excess scar tissue and/or fibrosis, for example ocular wound scarring in an animal.

The respective compounds can be administered by any suitable means. If the host organism is a mammal, the compound may for example be administered parenterally or non-parenterally (enterally). In a typical embodiment of administering to a mammal, the application ensures delivery to the wound site. In one embodiment, the administration is topical. In other embodiments, the preparation of the compound can be administered orally, intravenously, intraocularly or by inhalation. Examples for preparations for a topical application are drops, gels and ointments, examples for an oral application are tablets, pills or drinking solutions, examples for preparations for intravenous administrations are injection or infusion solutions, examples of preparations for administration by inhalation are aerosol mixtures or sprays.

Suitable methods for drug delivery include colloid drug delivery systems like liposomes, microspheres, microemulsions, nanoparticles, nanocapsules, such as polyelectrolyte capsules, to name only a few. Depending on the route of administration, the pharmaceutical composition may require protective coatings. Pharmaceutical forms suitable for injectionable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol and the like and mixtures thereof.

Suitable methods for ocular drug delivery include, but are not limited to topical drops, ointments, and therapeutic lenses, soluble ocular drug inserts, collagen shields, liposomes, and transcorneal iontophoresis. A class of polymers used in ocular drug delivery methods are dendrimers. Dendrimers, synthetic spherical macromolecules named after their characteristic "tree-like" or dendritic branching around a central core, can be used. The branching structure makes them a useful vector capable of efficient drug and gene delivery.

In another embodiment a Pluronic^{®} based systemic delivery system, such as a Pluronic^{®} F127-based drug delivery system can be used. F127 is a non-ionic, hydrophilic polyoxyethylene-polyoxypropylene (POE-POP) block copolymer previously used for its surfactant and protein stabilizing properties. F127-based matrices are characterized by a phenomenon known as reverse thermogelation whereby they undergo a phase transition from liquid to gel upon reaching physiological temperatures. Therefore, formulations of F127 can be administered in liquid form at temperatures less than approximately 10°C, with conversion to semisolid gels at body temperature, thereby potentially acting as sustained release depots. Furthermore, proteins contained within the pluronic matrix at high concentrations have been shown to retain their native configuration. Such a system can be used to prepare sustained release formulations. Such deliver systems have been desribed previously, such as by Claire M. Coeshott, et al., 2004, Vaccine, vol.22, no.19, pp. 2396 or by Suketu D. Desai and James Blanchard, 2000, Drug Delivery, vol.7, pp.201.

In various embodiments, the composition may, in addition to the SPARC activity and/or amount modulating agent, comprise one or more additional pharmaceutically active drugs. Examples for such drugs include, but are not limited to antibiotics, antiinflammatory agents, antiangiogenencis and analgesics.

Several active ingredients used in combination may not necessarily bring out their joint therapeutic effect directly at the same time in the mammal to be treated. Thus, the corresponding composition may also be in the form of a medical kit or package containing the two ingredients in separate but adjacent repositories or compartments. In the latter context, each active ingredient may therefore be formulated in a way suitable for an administration route different from that of the other ingredient, e.g. one of them may be in the form of an oral or parenteral formulation whereas the other is in the form of an ampoule for intravenous injection or an aerosol.

The invention will be further illustrated in the following description, with reference to the drawings.

### EXAMPLES

The following examples serve as a means to illustrate the various embodiments and should not be construed as limiting the scope of protection of the various embodiments.

### Example 1: Cell culture

SPARC-null (SPARC-/-) mice were obtained from Dr Helen Sage (Benaroya Research Institute, Seattle, Washington DC, USA). Conjunctival fibroblasts obtained from WT and SPARC-/- mice were cultured as explants in culture medium consisting of DMEM supplemented with 10% FBS and Penicillin-Streptomycin (100 U/ml and 100 *µ*g/ml, respectively) at 37°C in a humidified incubator containing 5% CO₂. Fibroblasts that migrated out from the tissue were propagated in the same medium. To induce a fibrotic response, cells were stimulated with 2 ng/ml recombinant TGF-β2 (PeproTech Inc., NJ, USA) for 72 h before subjected to real-time PCR assay. All tissue culture reagents were obtained from Invitrogen Corp. (CA, USA) unless otherwise stated.

### Example 2: RNA isolation and expression

Total RNA was harvested using Trizol Reagent (Invitrogen Corp.) according to the manufacturers' recommendations. First-strand cDNA was synthesized with 500 ng total RNA extract and 1 µl of 50 ng/µl random hexamer primer (Invitrogen Corp.) using Superscript III reverse transcriptase (Invitrogen Corp.) according to manufacturer's instructions. Quantitative real-time PCR (qPCR) was performed in a total volume of 10 µl using 384-well microtiter plates. Each reaction consists of 1 µl of first-strand reaction product, 0.5 µl each of upstream and downstream primers (10 µM each), 4 µl of Power SYBR Green PCR Master Mix (Applied BioSystems, CA, USA) and 4 µl of DNase-RNase free distilled water (Sigma-Aldrich Corp., MO, USA). Amplification and analysis of cDNA fragments were carried out using the Roche LightCycler 480 System (Roche Diagnostics Corp, Indianapolis, USA). All PCR reactions were performed in triplicates. All mRNA levels were measured as CT threshold levels and normalized with the corresponding β-actin CT values. Values are expressed as fold increase over the corresponding values for WT. The primers used are shown in Table 1.

**Table 1. Primers used for real-time PCR**

| Gene | Forward primer, 5'-3' | Reverse primer, 5'-3' |
|---|---|---|
| β-actin | CCAACCGCGAGAAGATGA (SEQ ID NO:3) | CCAGAGGCGTACAGGGATAG (SEQ ID NO:4) |
| collagen I | | |
| Fibronectin | | ACCAACCTACGGATGACTCG (SEQ ID NO:8) |
| α-SMA | | ACAGAGTATTTGCGCTCCGAA (SEQ ID NO:10) |

### Example 3: Collagen Gel Cultures

Cultured mouse fibroblasts were trypsinized, washed twice with DMEM, and resuspended in FBS at a density of 1.35 x 10⁶ per ml. Type I collagen (5mg/ml, Sigma-Aldrich), 10x DMEM and cell suspension were mixed on ice in a volume ratio of 6:2.5:1.5 (final concentration of type I collagen, 3 mg/ml; final cell density, 2 x 10⁵ per ml). 0.25 ml of the collagen gel suspension was added to each well of a 24-well cell culture dish and incubated at 37 °C for 30 minutes for polymerization. Gels were then detached and the wells topped up with 0.5 ml of culture medium. Contraction quantifications at day-1, -3 and -5 were performed using the Kodak Image Station 4000R (Carestream Molecular Imaging, New Haven, USA). All experiments were performed in triplicate.

### Example 4: Zymography and Western Blot analysis

To determine MMP-2 activity, equal amounts of conditioned medium recovered from contracted collagen gels on day-5 were resolved on pre-cast 10% SDS-polyacrylamide slab gels containing 0.1% gelatin (Invitrogen Corp.). Following electrophoresis, gels were renatured in renaturing buffer (Invitrogen Corp.) for an hour and incubated with developing buffer (Invitrogen Corp.) overnight for maximum sensitivity. MMP activity was visualized by staining the gels with Simplyblue Safestain (Invitrogen Corp.) for 1 hour. Images were taken and analysed with the Kodak Image Station 4000R (Carestream Molecular Imaging., New Haven, USA).

For harvesting fibroblasts from the gels, gels were treated as described (Arora et al., Am. J. PathoL 1999, 154, 871-882). The dispersed cells were lysed with extraction buffer (20 mM Tris-buffer, pH 7.4, 150 mM NaCl, 1 mM EDTA, 0.5% (v/v) Triton X-100, 2 mM MgCl₂, 1 mM DTT) and protein content was assessed by the Coomassie Plus Protein Assay Reagent (Thermo Fisher Scientific Inc., IL, USA). Equal amounts of protein were electrophoresed on a 10% SDS gel, transferred to nitrocellulose filters and probed with the rabbit antibody for MT1-MMP (Abcam plc, Cambridge, UK) followed by a horseradish-peroxidase-conjugated second antibody and developed with SuperSignal West Femto Maximum Sensitivity Substrate (Thermo Fisher Scientific Inc., IL, USA). Blots were stripped and probed with a rabbit antibody for GAPDH (Santa Cruz Biotechnology, Inc., CA, USA) for comparison.

### Example 5: Mouse model of glaucoma filtration surgery

C57BL/6 (WT) and SPARC-/- mice were bred and maintained at the department of experimental surgery (Singapore General Hospital, Singapore). All experiments with animals were approved by the IACUC. Modified filtering surgery was performed by dissection of the conjunctiva exposing the sclera followed by limbal incision with a 31.5G cannula introduced from the limbus into the anterior chamber. This created a fistula draining the aqueous humour into the subconjunctival space. The wound is secured and closed at the limbus by a 10-0 (0.2 metric) Ethilon black monofilament nylon scleral suture (Ethicon, Inc.). Fucithalmic ointment (Leo Pharmaceutical Products) was instilled at the end of the procedure. Surgery was performed on at least eight WT and eight SPARC-/- eyes.

### Example 6: Detection and measurement of blebs

Careful slit lamp and OCT examinations on subconjunctival blebs were performed daily until postoperative day 3, after which the blebs were observed every 3 to 4 days until postoperative day 14. Slit lamp biomicroscopy was performed with the Nikon FS-3V zoom photo slit lamp (Nikon, Japan). Bleb survival and size of at least 8 eyes of each genotype were measured by OCT using the Visante™ OCT, Anterior Segment Imaging Model 1000 (Carl Zeiss Meditech Inc., Dublin, CA, USA) and the images were analysed using the Visante™ OCT software. *In vivo* confocal microscopy using the Heidelberg retina tomography HRT3 (Heidelberg Engineering GmbH, Germany) was performed for at least three eyes of either genotype. *In vivo* confocal images were analysed with the Heidelberg Eye Explorer version 1.5.1 software.

### Example 7: Histology and immunofluorescent analyses

Control unoperated or operated eyes recovered on day-14 post-surgery were fixed in 4% paraformaldehyde before processing and sectioning using the Microm HM550 (Zeiss). 5 µm sections were stained with hematoxylin and eosin to visualize tissue morphology. To assess the collagen matrix, Picrosirius red staining was performed as previously described (Junqueira et al., 1979). For immunofluorescent analysis, antibodies specific for SPARC (Santa Cruz Biotechnology, Inc., CA, USA), collagen (Abcam plc, Cambridge, UK), fibronectin (Abcam plc, Cambridge, UK) and MMP-2 (Abbiotec, LLC, CA, USA) were used. The primary antibodies were visualized using secondary antibodies conjugated to either Alexa Fluro 488 or Alexa Fluro 594 (Invitrogen). Sections were visualized using the FV1000 confocal microscope (Olympus, USA).

### Example 8: Transmission electron microscopy (TEM) analysis

TEM was performed as described previously (Mehta et al., Cornea 2008, 27, 722-726). For measurement of collagen diameter, at least ten randomly selected fields of view of either WT or SPARC-/- conjunctiva (magnification, 56,000x) were photographed. The fibril diameters were measured using Image J software.

### Example 9: Statistical analysis

Data are expressed as mean ± standard deviation (SD) where appropriate. The significance of differences among groups was determined by the two-tailed Student's t-test using the Microsoft Excel 5.0 software, with significance at a *P*<0.05.

### Example 10: Effect of SPARC deficiency on the production of ECM proteins and myofibroblast differentiation induced by TGF-β2

Excess production and deposition of ECM proteins induced by TGF-β is known to play an important role in the development of fibrosis (Branton MH & Kopp JB, Microbes Infect. 1999, 1:15, 1349-1365). To evaluate the response of SPARC-/- conjunctival fibroblasts to TGF-β2, the expressions of collagen I and Fibronectin were examined on RNA level using real-time PCR. In this analysis the primers indicated in Table 1 were employed.

Cells treated with TGF-β2 (2 ng/ml) for 72 h were measured for collagen I, Fibronectin or α-SMA mRNA abundance by real-time quantitative PCR. The results are shown in Figure 1.

SPARC depletion significantly affected collagen I expression. First of all, SPARC-/-fibroblasts appeared to intrinsically express considerably lower levels of collagen I than WT cells (0.16-fold of WT, *P*<0.05) (Fig. 1A). Secondly, collagen I expression in the SPARC-/- cells was not enhanced by TGF-β2 stimulation whilst WT conjunctival fibroblasts demonstrated a spike in collagen I expression (Fig. 1A). Similarly, the presence of TGF-β2 induced a marked up-regulation of Fibronectin expression in WT cells (1.5-fold, *P*<0.005, Fig. 1B). In contrast, SPARC-/- cells showed a negligible increase in Fibronectin mRNA expression when stimulated with TGF-β2 (Fig. 1B). Altogether these data imply that SPARC deficiency may alleviate the fibrotic phenotype by reducing the production of the ECM components collagen I and Fibronectin when TGF-β2 is present. Moreover, the lower level of endogenous collagen I expression implies that the healing response is intrinsically altered in SPARC-/- mice.

TGF-β is also an important inducer of epithelial-mesenchymal transition (EMT) *in vivo* and *in vitro* (Willis and Borok, Am. J. Physiol. Lung Cell Mol. Physiol., 2007, 293, 525-534). It is considered to be the major factor in the differentiation of fibroblasts into myofibroblasts because it is capable of up-regulating the expression of the EMT marker α-SMA in fibroblasts. To determine if SPARC depletion affects TGF-β2-induced myofibroblast differentiation in conjunctival fibroblasts, we measured the expression of α-SMA in SPARC-/- and WT fibroblasts. In both, WT and SPARC-/- cells, an induction of α-SMA mRNA expression was observed in the presence of TGF-β2 (Fig. 1C). The fact that a similar response was observed in SPARC-/- and WT fibroblasts suggests that SPARC is not involved in the regulation of TGF-β2-induced myofibroblast differentiation of conjunctival fibroblasts. TGF-β is known to regulate α-SMA expression via the induction of β-Catenin (Masszi et al., 2004) which is a deviation from the canonical TGF-β signaling pathway. This may explain the preservation of the α-SMA-inducing pathway even in the absence of SPARC assuming that SPARC acts primarily through the canonical pathway. Therefore SPARC may be involved in regulating constitutive collagen I expression by modulating growth factor activities and during tissue injury by acting as an essential adaptor in the canonical TGF-β signaling pathway. Alternatively, SPARC may be directly involved in regulating the expression of these genes since it has been shown to be associated with the nuclear matrix (Gooden et al., 1999).

### Example 11: Effect of SPARC deficiency on fibroblast contractility and MMP-2 activity

Contraction of newly formed granulation tissue by fibroblasts to bring together the wound edges is an essential process in wound healing (Clark, The Molecular and Cellular Biology of Wound Repair (Edited by Clark RFA) 1996, 3-50). To assess the effect of SPARC deficiency in wound contraction *in vitro,* the well-established three-dimensional collagen lattice contraction model system was utilized (Arora et al., Am. J. PathoL, 1999, 154, 871-882; Tamariz and Grinnell, Mol. Biol. Cell. 2002, 13, 3915-3929; Grinnell, J. Cell. Biol. 1994, 124, 401-404; Grinnell, Trends Cell Biol. 2003, 13, 264-269). The free-floating gel involves releasing a freshly polymerized fibroblast-containing collagen matrix from the culture dish and allowing it to float in culture medium. Since there is no exertion of an external mechanical load on the contraction, actin stress fibers are not involved in the contraction process (Ehrlich and Rajaratnam, Tiss. Cell 1990, 22, 407-417). WT or SPARC-/- fibroblasts were cultured in 3D collagen matrices and gel area was measured over 5 days. The results are shown in Figure 2 and Table 2.

Contraction, as represented by gel area expressed as a percentage of the initial area, was rapid within the first 24 h. SPARC-/- cells contracted more rapidly than WT cells in this initial period, with a mean reduction to 37% of initial gel size in SPARC-/- cells compared to 45% in WT cells (*P*<0.05, n=3, Fig. 2A). Subsequent to the initial 24 h, the rate of contraction decreased and notably, the difference in the rates of contraction was reversed between the SPARC-/- and WT cells from day 3 to day 5. As shown in Table 2, the rate of gel contraction between day 3 and day 5 was 2.7-fold slower for SPARC-/- cells in comparison to WT cells.

**Table 2. Comparison of the rate of collagen gel contraction from day-3 to -5 between WT and SPARC-/- mouse conjunctival fibroblasts**

| | Mean % of initial gel size on day 3 | Mean % of initial gel size on day 5 | Mean rate of contraction between day 3 and day 5 (% of initial gel size/day) | Fold slower in rate |
|---|---|---|---|---|
| WT | 35.84 ± 0.16 | 25.53 ± 1.70 | -5.16 ± 0.77* | |
| SPARC-/- | 32.83 ± 0.27 | 28.99 ± 0.65 | -1.92 ± 0.19* | 2.69 |

Values represent the mean % of initial gel sizes ± SD. The rate of contraction between day 3 and day 5 is evaluated as the difference in % of initial gel size between day 5 and day 3 over 2 days. The mean rate of contraction is the average of the rates of contraction of 3 independent experiments, each was performed in triplicate. The fold slower in rate is calculated based on mean rate of contraction from day 3 to day 5 of WT over SPARC-/- fibroblasts. **P* = 0.01.

Matrix metalloproteinases (MMPs) have previously been shown to be essentially required for fibroblast-mediated collagen matrix contraction and ECM remodelling (Daniels et al., Amer. J. Pathol. 2003, 163, 2043-2052). Therefore the activity of MMPs secreted by the mouse fibroblasts seeded in the contracted collagen matrices was examined. Conditioned media were collected from the contracting matrices on day 5 and evaluated by gelatin zymography. Bands corresponding to metalloproteinases MMP-9 as well as pro- and active MMP-2 were detected in all samples (Figure 2B). The level of active MMP-2 in conditioned media from the SPARC-/- cells was substantially reduced in comparison with that from the WT cells. This is in contrast to the level of MMP-9 activity which was not different between the two cell lines (Figure 2B). Hence, the inhibitory effect of SPARC depletion on MMP activity appears to be specific for MMP-2. Since MMP-2 activation is dependent on the formation of a trimolecular complex consisting of MT1-MMP, pro-MMP-2 and TIMP-2 (Sato et al., Nature 1994, 370, 61-65), experiments were proceeded to determine if the reduction in MMP-2 activity is due to an alteration in the expression of MT1-MMP. Indeed, a Western Blot analysis of the cell lysates derived from the contracted matrices after 5 days showed a clear reduction in MT1-MMP expression in the SPARC-/- fibroblasts compared to the WT cells (Figure 2C). It was further verified that the free-floating matrices contracted independent of the involvement of actin stress fibers by determining the RNA expression of α-SMA in the cells derived from the 5-day old gels. As shown in Figure 2D, SPARC-/- cells expressed equivalent amounts of α-SMA mRNA as WT cells cultured in the collagen gels. Hence, the slower rate of day 3 to day 5 collagen gel contraction conferred by the SPARC-/- cells is potentially due to a reduction in MT1-MMP and MMP-2 activities. The RNA level was determined by real-time PCR using the primers of Table 1.

### Example 12: Effect of SPARC deficiency on bleb survival in a murine model of glaucoma filtration surgery

To investigate the effect of SPARC depletion on functional wound maintenance, a murine model of glaucoma filtration surgery was created. This form of surgery is routinely performed in humans to lower the intraocular pressure (IOP) if topical medications alone provide suboptimal pressure control. In brief, the conjunctiva was surgically dissected to expose the underlying sclera. This was followed by an incision through the sclera into the anterior chamber of the eye to creating a fistula (Fig. 3A). This surgically created channel facilitates the outflow of aqueous humor from the anterior chamber out into the subconjunctival space, thereby effectively reducing the IOP. Filtration of fluid is obvious as a conjunctival blister (bleb) which is readily observed by slit lamp microscopy (Fig. 3B). The blebs in the day-2 wounds were visible as raised and swollen areas of the conjunctiva in both WT and SPARC-/- eyes (Fig. 3Biii and iv). However, by day-14 post-surgery, all the WT wounded eyes appeared to have lost the blebs as evidenced by the flattened conjunctiva (Fig. 3Bv), whereas the majority of SPARC-/- wounded eyes retained the blebs (Fig. 3Bvi).

Further analysis by optical coherence tomography (OCT) was performed to evaluate bleb size and bleb survival. OCT measurements on days-2, -7, -10 and -14 post-surgery confirmed the slit lamp observations (Fig. 4A). The bleb in a representative WT wounded eye can be observed to progressively reduce in size until its complete disappearance by day-14 (Fig. 4A). In contrast, the bleb in a representative SPARC-/-wounded eye remained visible on each of the indicated days for at least 14 days (Fig. 4A). In fact, the bleb sizes of the individual SPARC-/- mice (n=8) at all time points can be observed to be generally larger than the WT mice (n=8) on each of the corresponding days measured (Fig. 4B). While the bleb sizes generally progressively diminished with wound age regardless of the genotype, the decline was notably steeper for the WT counterpart. The difference in the blebs between the two genotypes was most prominent on day-14 when the mean bleb size of wounded SPARC-/- eyes was 82% of the previous day-2 size compared to that of only 20% in wounded WT eyes (Fig. 4B). As is obvious from Fig. 4B, the bleb sizes, even when expressed as percentages of the corresponding day-2 sizes, can vary significantly between individual eyes. Difficulties in regulating the discrepancies in the size of the wound during the surgical process as well as the variations in the wound response between individual mice are likely to contribute to this spread. To allow for quantification of bleb survival, a bleb size that is ≥50% of the day-2 bleb size was deemed as a functioning bleb, i.e. positive for bleb survival whereas a bleb size of <50% was considered as a failure and non-survival. The validity of this criterion was confirmed with slit lamp microscopy which produced a similar score for bleb survival or failure. Here, it was found that bleb survival was nil in WT eyes (0/8) and 87.5% in SPARC-/- eyes (7/8) on day-14 post-wounding (Fig. 4C). In summary, both slit lamp and OCT measurements indicated that SPARC-/- mice were better able to maintain bleb function and remained at ≥50% of its initial size compared toWT.

### Example 13: SPARC-/- conjunctiva exhibits a diffuse connective tissue layer and responds to wounding distinct from WT

The mouse conjunctiva is composed of an epithelial covering of two to four layers of keratinocytes resting loose collagenous connective tissue delicately attached to the underlying sclera. To visualize the conjunctiva in vivo, the day-14 eyes were examined by confocal microscopy. As shown in Fig. 5A, the normal WT conjunctiva is organized in a distinct outermost epithelial layer followed by a spongey subconjunctival space (stroma) juxtaposed posteriorly with the sclera. Surprisingly, the SPARC-/- subconjunctival space appeared considerably less optically dense in comparison to the WT conjunctiva (Fig. 5B). At day-14 post-wounding, the WT conjunctival space had collapsed into a compact and optically dense matrix lacking any filtering space for aqueous outflow (Fig. 5C). In contrast thereto, the wounded SPARC-/- subconjunctival space had expanded several folds at the site of the surgery and was filled with optically diffuse lacey material (Fig. 5D). The loosely organized composition of the matrix in the latter facilitates the filtration of aqueous fluid through the fistula and percolation through the stromal network.

### Example 14: SPARC-/- wounded conjunctiva exhibits deficient collagen deposition and scar formation

The physio-anatomical differences underlying the wound response between WT and SPARC-/- eyes were investigated. Therefore, the eyes were cryosectioned on day-14 post-wounding and subjected to histopathological analyses. First of all, the SPARC -/conjunctiva epithelium (Fig. 6Biii) was conspicuously thinner than that in the WT eye (Fig. 6Bi). In agreement with the *in vivo* confocal microscopy data presented in Fig. 5, the wounded day-14 WT conjunctiva was characterized by thick strands of fibrous material in the subconjunctival space (Fig. 6Bii). In contrast, the SPARC-/- bleb appeared to be vacuous and punctuated with thin, wispy strands of matrix material (Fig. 6Biv). These observations suggest that collagen deposition is likely to be altered in the SPARC-/- wounds.

To determine anomalies in collagen deposition in the SPARC-/- compared to WT conjunctiva, sirus red polarization microscopy was performed. It was observed that the thinner conjunctiva in the SPARC-/- mouse was associated with a corresponding thinner collagen layer (Fig. 6Diii), whilst the thicker WT conjunctiva contained densely staining collagen fibers (Fig. 6Di). At the 14 day-old wound site, the WT conjunctiva appeared to be densely compacted with thick, well-aligned collagen fibers resembling a scar (Fig. 6Dii). This arrangement differs from the mechanically efficient basketweave meshwork of collagen in normal conjunctiva (Fig. 6Di). On the other hand, the day-14 bleb in the SPARC-/- eyes contained thin and loosely assembled collagen fibers in an expanded non-collagenous subconjunctival space (Fig. 6Div). Ultrastructural analysis confirmed that the WT bleb consisted of tightly packed collagen fibrils (Fig. 6Ei) which became even more densely packed with the absence of any gaps between fibers (Fig. 6Eii). In great contrast, the SPARC-/- conjunctiva features loosely organized fibers (Fig. 6Eiii) which became pronouncedly diffuse and interspersed with numerous non-fibrous areas in the bleb (Fig. 6Eiv). In addition, a measurement of the collagen fibrils indicated that the WT bleb possessed thicker collagen fibrils (Fig. 6Ei, inset) with a mean fibril diameter of 122.72 nm compared to SPARC-/- conjunctiva which contained two-fold thinner collagen fibrils (Fig. 6Eiii, inset) with a mean diameter of 59.50 nm (Fig. 6F). Thus, from these observations it appears that the prolonged bleb survival in the SPARC-/- eyes is related, at least in part, to the deposition of thinner, fewer and loosely organized collagen fibers. Conversely, the seemingly flattened blebs found in the WT eyes that consisted of dense, parallel bundles of collagen fibers is reminiscent of scar formation and is likely to be the cause of fistula obstruction with impediment of aqueous outflow leading to bleb failure.

### Example 15: Expression of ECM components is generally suppressed in SPARC-/- wounds

To determine the expression of ECM components in the wounds, immunofluorescence analyses of the normal and wounded mouse conjunctiva were performed. In agreement with previous observations that SPARC expression is up-regulated at sites of injury, substantial SPARC expression in the wounded WT conjunctiva was detected (Fig. 7B), which contrasted dramatically the low level expression detected in normal conjunctiva (Fig. 7A). An immunostaining for collagen I confirmed the sirius red staining data illustrated in Fig. 6. The WT blebs (Fig. 7F) demonstrated intense collagen I expression in comparison to its normal counterpart (Fig. 7E). Notably, the collagen I immunoreactivity was profoundly diminished in the SPARC-/- eyes (Fig. 7G) with the wounding failing to incite an increase in expression (Fig. 7H). Fibronectin expression was also observed to be greatly up-regulated when WT conjunctiva was wounded (Fig. 7J) compared to the normal conjunctiva where it was barely detectable (Fig. 7J). In the SPARC-/- conjunctiva, wounding resulted in only a slight up-regulation in Fibronectin expression (Fig. 7L) compared to its unwounded counterpart (Fig. 7K). Finally, an examination for MMP-2 revealed its markedly enhanced expression in WT conjunctiva upon wounding (Fig. 7N), whereas in the injured SPARC-/- conjunctiva the elevation of its expression was minimal (Fig. 7P).

### Example 16: In vitro modulation of SPARC expression

The subconjunctival tenon fibroblast is the main effector cell that drives the wound healing process following conjunctival injury. In order to investigate the possibility to modulate the SPARC expression and thereby allowing to modulate extracellular membrane proteins, human tenon fibroblast cells were transfected siRNA. To knockdown SPARC, the following small interfering RNA (si-SPARC) was used: 5'-AACAAGACCUUCGACUCUUCCC-3' (SEQ ID NO:2). A nonsilencing scrambled control (si-Scram) was also used: 5'-GCUCACAGCUCAAUCCUAAUC-3' (SEQ ID NO:11). HTFs were transfected with 100 nM SPARC siRNA using Lipofectamine 2000 (Invitrogen Corp., Carlsbad, CA) according to manufacturer's instructions. HTFs transfected with si-SPARC showed efficient silencing of SPARC mRNA expression by 99.98% relative to that in HTFs transfected with the negative control si-Scram 24 hours after transfection. This level of inhibition was sustained for at least one week, as shown by quantitative RT-PCR (qPCR) (Figure 14). After 7 days the expression of SPARC was also examined on protein level by Western Blot analysis with anti-SPARC and anti-β-tubulin (loading control) antibodies. The Western Blot analysis confirmed that SPARC protein expression was dramatically reduced in the presence of si-SPARC (Figure 8a). The significant knockdown of the SPARC gene product is evident and is comparable to the absence of SPARC observed in mouse fibroblast cells derived from a SPARC -/- genetic knockout mouse (Figure 8b).

### Example 17: Duration of the SPARC knockdown in siRNA transfected human tenon's fibroblast cells

According to the protocol detailed in Example 7, human tenon's fibroblast cells were transfected once with either si-SPARC or si-Scram and maintained in collagen gels. Using real-time PCR the amount of SPARC was quantified (for Primers see Table 1). Mitomycin C (MMC) (0.1 to 0.4 mg/ml) is sometimes used in the treatment of wounds against risk factors for excess scar formation (see Background of the invention). In order to study, whether this drug exhibits its beneficial effects via a modulation of the SPARC expression, MMC was also included in these studies. As in Example 11, these experiments were performed with cells in collagen gels. The analyses revealed that on the first day the amount of SPARC RNA transcripts is strongly reduced in cells transfected with si-SPARC, whereas the control cells transfected with si-Scram did not shown an altered expression In both cases, the addition of MMC in the concentration of 01-0.4 mg/ml) did not significantly change the SPARC expression (Figure 9 and Figure 10). The analysis was repeated after 3 and 7 days. On both days, the amount of SPARC was strongly reduced when analyzing si-SPARC transfected cells, whereas si-Scram transfected cells in presence or absence of MMC did not show a significantly modulated SPARC level (Figure 11 and Figure 12). On the seventh day a Western Blot analysis was performed to confirm the results on protein expression levels. As Figure 13 shows, the SPARC protein level were strongly reduced 7 days after a single transfection with si-SPARC, whereas the cells transfected with si-Scram showed large amounts of SPARC, no matter whether MMC was also added to the cell culture.

### Example 18: SPARC knockdown in siRNA transfected mouse subjunctival fibroblast cells

To verify that SPARC expression in mouse subconjunctival fibroblasts can be similarly silenced with siRNA as in human tenon's fibroblast cells, parallel knockdown experiments on mouse cells were performed. Mouse conjunctival fibroblasts were transfected with si-SPARC or si-Scram as described above and maintained in culture for up to 7 days. As for the human cells (Example 17) the SPARC RNA level was examined by quantitative real time-PCR using the primers of Table 1. Figure 15 represents the data obtained after 1 or 7 days of transfection. From the data it becomes apparent that from the first up to at least the seventh day after transfection a reduction of the SPARC RNA level is achieved using si-SPARC, whereas si-Scram has no effect. Hence, similar as for the human cells, a sustained reduction of SPARC mRNA expression was achieved in mouse cells.

## Claims

1. A pharmaceutical composition for use in the prevention of post operative wound scarring after glaucoma filtration surgery or preserving a surgically-induced functional wound after glaucoma filtration surgery, wherein the composition is capable of reducing the amount and/or activity of SPARC protein (secreted protein acidic and rich in cysteine).

2. The pharmaceutical composition for use according to claim 1,
wherein the amount of the SPARC protein is reduced in tissue surrounding a wound site.

3. A pharmaceutical composition for use according to claim 1 wherein the composition modulates the production of at least one extracellular membrane (ECM) component, comprising reducing the amount and/or the activity of SPARC protein.

4. The pharmaceutical composition for use according to claim 3, wherein the at least one extracellular membrane (ECM) component is an ECM protein,
wherein the ECM protein is optionally collagen I and/or fibronectin.

5. The pharmaceutical composition for use according to any one of claims 1 to 4,
wherein the amount and/or activity of SPARC protein is reduced by reducing the expression of the nucleotide sequence encoding the SPARC protein.

6. The pharmaceutical composition for use according to claim 4,
wherein when the amount and/or activity of SPARC protein is reducd by reducing the expression of the nucleotide sequence encoding the SPARC protein, reducing the expression of the nucleic acid sequence encoding for the SPARC protein is optionally carried out by means of a nucleic acid molecule, wherein the nucleic acid molecule is optionally a non-coding nucleic acid molecule, the non-coding nucleic acid molecule optionally being RNA or DNA, the RNA or DNA optionally being selected from the group consisting of an DNA or RNA aptamer, an anti-sense-RNA molecule, a silencer-RNA molecule, a short hairpin RNA (shRNA) molecule, a micro RNA (miRNA) molecule, a small interfering RNA (siRNA) molecule and a repeat-associated small interfering RNA (rasiRNA) molecule.

7. The pharmaceutical composition for use according to claim 6, wherein the nucleic acid molecule is siRNA, wherein the siRNA optionally comprises or consists of the nucleotide sequence 5'-AACAAGACCUUCGACUCU UCCC-3'.

8. The pharmaceutical composition for use according toany one of claims 1 to 4, wherein reducing the amount and/or activity of SPARC comprises the use of a SPARC amount and/or activity reducing agent selected from the group consisting of a peptide, a polypeptide, a peptoid, an inorganic molecule and a small organic molecule.

9. The pharmaceutical composition for use according to claim 8,
(a) wherein the polypeptide is selected from the group consisting of an antibody, a fragment of an antibody, and a proteinaceous binding molecule with antibody-like functions, or
(b) wherein the peptide is an aptamer.

10. The pharmaceutical composition for use according to any one of claims 1 to 9, wherein the use of the composition comprises reducing the amount and/or activity of SPARC protein in a subject in need thereof,
(a) wherein the composition is administerable as a therapeutically effective amount comprising an agent that reduces the amount and/or activity of a SPARC protein to the subject, or
(b) wherein the subject is optionally an animal, optionally a mammal, or optionally selected from the group consisting of a rat, a mouse, a rabbit, a guinea pig, an opossum, a squirrel, a nine-banded armadillo, a dog, a cat, an elephant, a chimpanzee, a rhesus monkey, a macaque, an orangutan, a cattle (cow), a marmoset, an American pika, a galago and a human.

11. The pharmaceutical composition for use according to claim 1, wherein the wound is a surgically placed fistula.

12. The pharmaceutical composition for use according to any one of claims 1 to 11, wherein the method is used in a treatment for lowering intraocular pressure.

13. The pharmaceutical composition for use according to any one of claims 1 to 12,
(a) wherein the pharmaceutical composition is in the form of liquid drops, an ointment, a gel, a therapeutic lens, a soluble ocular drug insert, a collagen shield; and/or
(b) wherein the composition is encapsulated in liposomes, polyelectrolyte capsules, or biodegradable polymeric carriers; and/or
(c) wherein the composition further comprises one or more excipients; and/or
(d) wherein reducing the formation of excess scar tissue and/or fibrosis comprises inhibiting ocular wound scarring.

## Patentansprüche

1. Eine pharmazeutische Zusammensetzung zur Verwendung bei der Prävention von post-operativer Wundvernarbung nach filtrierender Glaukomchirurgie oder Erhaltung einer chirurgisch induzierten funktionalen Wunde nach filtrierender Glaukomchirurgie, wobei die Zusammensetzung in der Lage ist die Menge und/oder Aktivität von SPARC Protein (secreted protein acidic and rich in cysteine) zu verringern.

2. Die pharmazeutische Zusammensetzung zur Verwendung gemäß Anspruch 1, wobei die Menge des SPARC Proteins in dem Gewebe, das die Wunde umgibt, verringert ist.

3. Eine pharmazeutische Zusammensetzung zur Verwendung gemäß Anspruch 1, wobei die Zusammensetzung die Herstellung von mindestens einer extrazelluläre Membran (ECM) Komponente moduliert, umfassend das Reduzieren der Menge und/oder der Aktivität des SPARC Proteins.

4. Die pharmazeutische Zusammensetzung zur Verwendung gemäß Anspruch 3, wobei die mindestens eine extrazelluläre Membran (ECM) Komponente ein ECM Protein ist, wobei das ECM Protein optional Kollagen I und/oder Fibronectin ist.

5. Die pharmazeutische Zusammensetzung zur Verwendung gemäß einem der Ansprüche 1 bis 4, wobei die Menge und/oder Aktivität des SPARC Proteins reduziert ist durch Verringern der Expression der Nukleotidsequenz, die für das SPARC Protein kodiert.

6. Die pharmazeutische Zusammensetzung zur Verwendung gemäß Anspruch 4, wobei die Menge und/oder Aktivität des SPARC Proteins reduziert ist durch Verringern der Expression der Nukleotidsequenz, die für das SPARC Protein kodiert, wobei das Verringern der Expression der Nukleotinsäuresequenz, die für das SPARC Protein kodiert, optional mittels eines Nukleinsäuremoleküls erreicht wird, wobei das Nukleinsäuremolekül optional ein nicht-kodierendes Nukleinsäuremolekül ist, wobei das nicht-kodierende Nukleinsäuremolekül optional RNA oder DNA ist und die RNA oder DNA optional aus einer Gruppe ausgewählt ist, die aus einem DNA oder RNA Aptamer, einem anti-sense RNA Molekül; einem silencer-RNA Molekül, einem short hairpin RNA (shRNA) Molekül, einem mikro RNA (miRNA) Molekül, einem kurzen interferierenden RNA (siRNA) Molekül und einem Repeat-assoziierten kurzen interferierenden RNA (rasiRNA) Molekül besteht.

7. Die pharmazeutische Zusammensetzung zur Verwendung gemäß Anspruch 6, wobei das Nukleinsäuremolekül siRNA ist, wobei die siRNA optional die Nukleotidsequenz 5'-AACAAGACCUUCGACUCUUCCC-3' umfasst oder daraus besteht.

8. Die pharmazeutische Zusammensetzung zur Verwendung gemäß einem der Ansprüche 1 bis 4, wobei das Verringern der Menge und/oder Aktivität von SPARC die Verwendung von einem SPARC Menge- und/oder Aktivitäts-reduzierenden Wirkstoff umfasst, ausgewählt aus der Gruppe bestehend aus einem Peptid, einem Polypetid, einem Peptoid, einem anorganische Molekül und einem kleinen organischen Molekül.

9. Der pharmazeutischen Zusammensetzung zur Verwendung gemäß Anspruch 8,
(a) wobei das Polypeptid aus der Gruppe ausgewählt ist, die aus einem Antikörper, einem Fragment eines Antikörpers und einem proteinartigen Bindungsmolekül mit Antikörper-ähnlichen Funktionen besteht, oder
(b) wobei das Peptid ein Aptamer ist.

10. Die pharmazeutische Zusammensetzung zur Verwendung gemäß einem der Ansprüche 1 bis 9, wobei die Verwendung der Zusammensetzung das Verringern der Menge und/oder der Aktivität des SPARC Proteins in einem Subjekt, das dessen bedarf, umfasst,
(a) wobei die Zusammensetzung in einer therapeutisch wirksamen Menge verabreichbar ist, umfassend einen Wirkstoff, der die Menge und/oder die Aktivität eines SPARC Proteins in einem Subjekt reduziert, oder
(b) wobei das Subjekt optional ein Tier, optional ein Säugetier oder optional ausgewählt ist, aus der Gruppe bestehend aus einer Ratte, einer Maus, einem Kaninchen, einem Meerschweinchen, einem Opossum, einem Eichhörnchen, einem Neunbinden-Gürteltier, einem Hund, einer Katze, einem Elefant, einem Schimpansen, einem Rhesusaffen, einem Makaken, einem Orang-Utan, einem Rind (Kuh), einem Seidenaffen, einem Amerikanischen Pika, einem Galago und einem Menschen.

11. Die pharmazeutische Zusammensetzung zur Verwendung gemäß Anspruch 1, wobei die Wunde eine chirurgisch platzierte Fistel ist.

12. Die pharmazeutische Zusammensetzung zur Verwendung gemäß einem der Ansprüche 1 bis 11, wobei das Verfahren in der Behandlung zur Senkung des Augeninnendrucks verwendet wird.

13. Die pharmazeutische Zusammensetzung zur Verwendung gemäß einem der Ansprüche 1 bis 12,
(a) wobei die pharmazeutische Zusammensetzung in Form von flüssigen Tropfen, einer Salbe, einem Gel, einer therapeutischen Linse, einer löslichen okularen Medikamenteneinlage, einem Kollagenschild vorliegt; und/oder
(b) wobei die Zusammensetzung in Liposomen, Polyelektrolyt-Kapseln oder biologisch abbaubaren polymeren Trägern eingeschlossen ist; und/oder
(c) wobei die Zusammensetzung ferner einen oder mehrere Trägerstoffe umfasst; und/oder
(d) wobei das Reduzieren der Bildung von überschüssigem Narbengewebe und/oder Fibrose das Hemmen der Verarbeitung von okularen Wunden umfasst.

## Revendications

1. Composition pharmaceutique pour son utilisation dans la prévention de la cicatrisation d'une plaie postopératoire après une chirurgie de filtration de glaucome ou dans la conservation d'une plaie fonctionnelle induite par voie chirurgicale après une chirurgie de filtration de glaucome, dans laquelle la composition est capable de réduire la quantité et/ou l'activité de la protéine SPARC (secreted protein acidic and rich in cysteine ou protéine sécrétée acide et riche en cystéine).

2. Composition pharmaceutique pour son utilisation selon la revendication 1, dans laquelle la quantité de la protéine SPARC est réduite dans le tissu entourant un site de plaie.

3. Composition pharmaceutique pour son utilisation selon la revendication 1, dans laquelle la composition module la production d'au moins un composant de membrane extracellulaire (ECM), comprenant la réduction de la quantité et/ou de l'activité de la protéine SPARC.

4. Composition pharmaceutique pour son utilisation selon la revendication 3, dans laquelle l'au moins un composant de membrane extracellulaire (ECM) est une protéine ECM,
dans laquelle la protéine ECM est facultativement le collagène I et/ou la fibronectine.

5. Composition pharmaceutique pour son utilisation selon l'une quelconque des revendications 1 à 4,
dans laquelle la quantité et/ou l'activité de la protéine SPARC sont réduites par réduction de l'expression de la séquence de nucléotides codant pour la protéine SPARC.

6. Composition pharmaceutique pour son utilisation selon la revendication 4,
dans laquelle lorsque la quantité et/ou l'activité de la protéine SPARC sont réduites par réduction de l'expression de la séquence de nucléotides codant pour la protéine SPARC, la réduction de l'expression de la séquence d'acide nucléique codant pour la protéine SPARC est facultativement réalisée au moyen d'une molécule d'acide nucléique, dans laquelle la molécule d'acide nucléique est facultativement une molécule d'acide nucléique non codante, la molécule d'acide nucléique non codante étant facultativement de l'ARN ou de l'ADN, l'ARN ou l'ADN étant facultativement sélectionné dans le groupe constitué d'un aptamère d'ADN ou d'ARN, d'une molécule d'ARN antisens, d'une molécule d'ARN silencieux, d'une molécule d'ARN court en épingle à cheveux (ARNsh), d'une molécule de microARN (miARN), d'une molécule de petit ARN interférent (ARNsi) et d'une molécule de petit ARN interférent associé à des répétitions (ARNrasi).

7. Composition pharmaceutique pour son utilisation selon la revendication 6, dans laquelle la molécule d'acide nucléique est l'ARNsi, dans laquelle facultativement l'ARNsi comprend ou est constitué de la séquence de nucléotides 5'-AACAAGACCUUCGACUCU UCCC-3'.

8. Composition pharmaceutique pour son utilisation selon l'une quelconque des revendications 1 à 4, dans laquelle la réduction de la quantité et/ou de l'activité de SPARC comprend l'utilisation d'un agent réduisant la quantité et/ou l'activité de SPARC sélectionné dans le groupe constitué d'un peptide, d'un polypeptide, d'un peptoïde, d'une molécule inorganique et d'une petite molécule organique.

9. Composition pharmaceutique pour son utilisation selon la revendication 8,
(a) dans laquelle le polypeptide est sélectionné dans le groupe constitué d'un anticorps, d'un fragment d'un anticorps, et d'une molécule de liaison protéique avec des fonctions de type anticorps, ou
(b) dans laquelle le peptide est un aptamère.

10. Composition pharmaceutique pour son utilisation selon l'une quelconque des revendications 1 à 9, dans laquelle l'utilisation de la composition comprend la réduction de la quantité et/ou de l'activité de la protéine SPARC chez un sujet le nécessitant,
(a) dans laquelle la composition peut être administrée au sujet comme une quantité thérapeutiquement efficace comprenant un agent qui réduit la quantité et/ou l'activité d'une protéine SPARC, ou
(b) dans laquelle le sujet est facultativement un animal, facultativement un mammifère, ou facultativement sélectionné dans le groupe constitué d'un rat, d'une souris, d'un lapin, d'un cobaye, d'un opossum, d'un écureuil, d'un tatou à neuf bandes, d'un chien, d'un chat, d'un éléphant, d'un chimpanzé, d'un singe rhésus, d'un macaque, d'un orang-outan, de bétail (d'une vache), d'un ouistiti, d'un pika américain, d'un galago et d'un être humain.

11. Composition pharmaceutique pour son utilisation selon la revendication 1, dans laquelle la plaie est une fistule créée chirurgicalement.

12. Composition pharmaceutique pour son utilisation selon l'une quelconque des revendications 1 à 11, dans laquelle le procédé est utilisé dans un traitement de réduction de la pression intraoculaire.

13. Composition pharmaceutique pour son utilisation selon l'une quelconque des revendications 1 à 12,
(a) dans laquelle la composition pharmaceutique se trouve sous la forme de gouttes liquides, d'un onguent, d'un gel, d'une lentille thérapeutique, d'un insert oculaire soluble contenant un médicament, d'un écran en collagène ; et/ou
(b) dans laquelle la composition est encapsulée dans des liposomes, des capsules de polyélectrolytes, ou des supports polymères biodégradables ; et/ou
(c) dans laquelle la composition comprend en outre un ou plusieurs excipients ; et/ou
(d) dans laquelle la réduction de la formation d'un excès de tissu cicatriciel et/ou de fibrose comprend l'inhibition de la cicatrisation d'une plaie oculaire.
